(19) Europäisches Patentamt
European Patent Office
Office européen des brevets

(11) **EP 2 321 109 B1**

(12) **EUROPÄISCHE PATENTSCHRIFT**

(45) Veröffentlichungstag und Bekanntmachung des Hinweises auf die Patenterteilung:
**14.10.2020 Patentblatt 2020/42**

(21) Anmeldenummer: **09777463.2**

(22) Anmeldetag: **27.07.2009**

(51) Int Cl.:
*F16L 11/04* (2006.01)  *B29C 49/48* (2006.01)
*B29C 33/04* (2006.01)  *B29C 49/14* (2006.01)
*A61M 25/10* (2013.01)  *B29C 35/04* (2006.01)
*B29C 49/04* (2006.01)  *B29C 55/28* (2006.01)
*A61M 39/08* (2006.01)

(86) Internationale Anmeldenummer:
**PCT/EP2009/005430**

(87) Internationale Veröffentlichungsnummer:
**WO 2010/009901 (28.01.2010 Gazette 2010/04)**

(54) **VORRICHTUNG UND VERFAHREN ZUR HERSTELLUNG VON BALLON- ODER HOHLKÖRPERN AUS EINEM SCHLAUCHFÖRMIGEN ROHLING ODER EXTRUDAT DURCH BLASFORMUNG**

DEVICE AND METHOD FOR PRODUCING BALLOON-SHAPED BODIES OR HOLLOW BODIES FROM A TUBULAR BLANK OR EXTRUDATE BY BLOW MOULDING

DISPOSITIF ET PROCÉDÉ DE PRODUCTION DE CORPS DE BALLONNET OU DE CORPS CREUX À PARTIR D'UNE ÉBAUCHE OU D'UN EXTRUDAT TUBULAIRE PAR MOULAGE PAR SOUFFLAGE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **25.07.2008 DE 102008035023**
**25.07.2008 DE 102008035024**
**13.08.2008 DE 102008038954**
**24.11.2008 DE 102008058762**
**21.02.2009 DE 102009010038**

(43) Veröffentlichungstag der Anmeldung:
**18.05.2011 Patentblatt 2011/20**

(73) Patentinhaber: **Creative Balloons GmbH**
**68753 Waghäusel (DE)**

(72) Erfinder: **GÖBEL, Fred**
**69259 Wilhelmsfeld (DE)**

(74) Vertreter: **Küchler, Stefan**
**Patentanwalt**
**Färberstrasse 20**
**90402 Nürnberg (DE)**

(56) Entgegenhaltungen:
EP-A- 1 142 689    US-A- 2 222 461
US-A- 4 162 700    US-A- 5 993 721
US-A1- 2005 240 303    US-B1- 7 168 942

• DATABASE WPI Week 199303 Thomson Scientific, London, GB; AN 1993-023427 XP002584981 -& JP 04 351526 A (UBE IND LTD) 7. Dezember 1992 (1992-12-07)
• EICHLER T: "BERUEHRUNGSLOSE TEMPERATURMESSUNG IN DER KUNSTSTOFFINDUSTRIE SCHNELL UND GENAU" PLASTVERARBEITER, HUETHIG GMBH, HEIDELBERG, DE, Bd. 52, Nr. 4, 1. April 2001 (2001-04-01), Seite 94/95, XP001036332 ISSN: 0032-1338
• HOHENGASSNER H: "TRANSPARENZ IM THERMOFORMPROZESS DURCH ERWEITERUNG DES STEUERUNGSSYSTEMS" KUNSTSTOFFBERATER, 201975 1, Bd. 37, Nr. 5, 1. Mai 1992 (1992-05-01), Seiten 40-42, XP000293717 ISSN: 0172-6374

**Beschreibung**

Die Erfindung richtet sich auf eine Vorrichtung und ein Verfahrengemäß den Ansprüchen 1 und 10 zur Herstellung von Ballon- oder Hohlkörpern . Für das Blasformen von Ballons oder Hohlkörpern, insbesondere für die Anwendung auf medizinischen Kathetern oder Geräten, sind derzeit im Wesentlichen zwei verschiedene Verfahren bekannt: Zum einen das sog. Ein-Schritt- oder In-line-Verfahren, wobei der Ballon direkt aus der zum Schlauch extrudierten, noch geschmolzen verformbaren Kunststoffmasse in ein Formwerkzeug geblasen wird. Die dabei entstehenden Ballonkörper weisen in der Regel einen wenig orientierten, überwiegend amorphen Materialzustand auf. Auf Grund der nur geringfügigen polymeren Orientierung sind im In-line-Verfahren hergestellte Katheterballon-Komponenten in der Regel nur wenig mechanisch belastbar, und für viele Applikationen, vor allem solche, die hohe Belastungsdrucke, Zugkräfte, geometrische Stabilität über lange Anwendungsintervalle oder zuverlässig einstellbare Ballon-Arbeitsdurchmesser verlangen, nur bedingt oder nicht geeignet.

Besonders problematisch wirkt sich der weitgehend amorphe Zustand der Ballonhülle bei sehr dünnwandigen Ballons, im Mikrometerbereich, aus. Die mechanische Beständigkeit solcher ausgeformten Hüllen ist bei Verwendung fast aller gängigen Ballonpolymere in der Regel ungenügend bzw. verursacht schon während der Ausformung des Ballons auf die erforderlichen niedrigen Wandstärken erhebliche Probleme.

Entscheidend beim qualitativ und quantitativ überlegenen Blasformen dünnwandiger Ballonfolien ist eine möglichst homogene, simultane Erwärmung sämtlicher Segmente bzw. Oberflächen eines Formwerkzeuges. Um dies zu erreichen, geht die vorliegende Erfindung von der Verwendung flüssiger Temperierungsmedien mit hoher Wärmetransportkapazität aus, und verzichtet bewusst auf andere Techniken, wie z.B. elektrische Heizkomponenten, induktionstechnische Verfahren oder die Erwärmung mit heißen Gasen.

Die Formwerkzeuge bestehen beim In-line-Verfahren in der Regel aus mehr oder weniger massiven Metallblöcken, die im Inneren die Negativkontur des zu formenden Körpers abbilden. Das Werkzeug wird entweder direkt durch im Metallblock integrierte Kanäle bzw. dort eingebaute Vorrichtungen zur Wärmeentwicklung und - abführung temperiert, oder durch eine das Werkzeug umschließende separate Heiz- und Kühlvorrichtung (Temperierungsmanschette), erhitzt oder gekühlt. Zur Erreichung eines ausreichenden Energieflusses wird im jeweiligen temperierenden Element in der Regel auf ein Temperaturniveau erwärmt oder gekühlt, welches deutlich über oder unter dem liegt, das an der formenden Grenzfläche zum Ballon erreicht werden soll.

Das an der formenden Grenzfläche errichte Temperierungsniveau wird in der Regel punktuell über im Werkzeugmantel oder in der Temperierungsmanschette angeordnete Temperatürfühler erfasst oder zumindest annähernd ermittelt, und als Messwert der Temperierungsregelung zugeführt.

Bei höheren Belastungsanforderungen an den Ballon oder Hohlkörper wird in der Regel das sog. Zwei-Schritt-Verfahren angewendet, wobei im ersten Fertigungsschritt zunächst ein Rohschlauch vorextrudiert und gelagert wird. In einem zweiten Schritt wird der Rohschlauch in ein Blasformwerkzeug eingeführt, optional dort vorgewärmt, anschließend über seine Längsachse gestreckt, und dann mit in das Schlauchlumen gepresster Druckluft in das Formwerkzeug hinein aufgebläht. Der Forminnenwand anliegend wird die Ballonhülle auf eine zur vollständigen Ausformung geeignete Temperatur erhitzt, so dass sich die in der Wandung des Ballons entwickelnden Retraktionskräfte entspannen und die durch die angewandte axiale und radiale Streckung des Materials erreichte Parallelisierung der Polymerketten in Streckrichtung nach anschließender Abkühlung der Ballonhülle fixiert wird.

Die erreichte räumliche Orientierung der Polymerketten im Sinne einer Parallelisierung, stattet den entstehenden Ballonkörper mit hoher mechanischer Belastbarkeit, insbesondere Zugfestigkeit in Richtung der Kettenorientierung aus. Bereits während des Blasformvorganges erreicht die Ballonhülle einen Grad an Stabilität, dass selbst geringste Wandstärken zuverlässig ausgeformt werden können.

Die ballonausformenden Module derzeit verwendeter Zwei-Schritt-Blasformungs-Maschinen bestehen in der Mehrzahl aus einer äußeren Heiz- und Kühleinrichtung mit einer hohlzylindrischen Ausnehmung in der Mitte zur Aufnahme des eigentlichen Blasformwerkzeuges. Dieses hat einen dazu komplementären, zylindrischen Außenumfang, so dass es passgenau in die Ausnehmung der Heiz- und Kühleinrichtung eingesetzt werden kann. Die Blasformwerkzeüge haben eine innere Ausnehmung, deren Gestalt der gewünschten Ballonform entspricht; diese Gestalt ist in einen ansonsten massiven Block eingeschnitten bzw. eingedreht.

Durch ihren massiven, massereichen Aufbau weisen diese Formen in der Regel, ähnlich wie beim Ein-Schritt-Verfahren, eine sehr hohe Wärmekapazität auf und benötigen daher lange, bis sie eine gewünschte Temperatur durch eine entsprechende Beheizung/Kühlung von außen erreicht haben.

Vor allem bei der Ausformung von Ballonkörpern mit einer großen Differenz der Durchmesser der auszuformenden extrudierten Rohschlauchmasse (Einschritt-Verfahren) bzw. des eingeführten vor-extrudierten Rohschlauches (Zweischritt-Verfahren) sowie des größten bei der Formung zu erreichenden Ballondurchmessers können die resultierenden Formwerkzeugmassen für eine wirtschaftliche, zykluszeitorientierte Fertigung problematisch werden.

Hohe Formwerkzeugmassen sind ebenfalls die Regel, wenn größere, komplex geformte Hohlkörperstrukturen mit deutlich von einander abweichenden Abmessungen der einzelnen Ballonsegmente auszuformen sind.

Um den Energie-Einstrom in das Formwerkzeug hinein oder die Abführung von Energie aus ihm heraus bei solchen massereichen Formen zu beschleunigen, wird zwischen der die Energie in das Formwerkzeug einbringenden und abführenden Heiz- und Kühlvorrichtung sowie dem darin eingebrachten Formwerkzeug in der Regel ebenfalls ein Temperaturgradient eingestellt, der die im Werkzeug für den jeweiligen Schritt innerhalb des Blasvorganges erforderliche Formtemperatur deutlich überschreitet (bei der Erwärmung) oder unterschreitet (bei der Kühlung). Nur so lässt sich ein zykluszeit-verkürzender, ausreichend schneller Wärmefluss erreichen.

Ähnlich einer ballistischen Kurve wird über die Heiz- und Kühlvorrichtung bei der Temperierung der einzelnen Formungsschritte im Formwerkzeug eine initiale Wärmemenge in das Formwerkzeug eingebracht, die dann in einem folgenden Ruheintervall, ohne weitere äußere Zufuhr von Wärmeenergie das Werkzeug gleichförmig durchwärmt und im Plateaubereich der sich einstellenden Temperaturkurve schließlich der gewünschten Arbeitstemperatur nähert. Der vor der Durchwärmung des Werkzeuges durch Wärmezufuhr oder -abfuhr eingestellte initiale Gradient darf jedoch nicht dazu führen, dass das sich im Formwerkzeug schließlich einstellende Temperaturniveau von der jeweils gewünschten . Formtemperatur des Kunsstoffes abweicht. Bei diesem "ballistischen" Verfahren besteht daher die grundsätzliche Schwierigkeit darin, das gewünschte Temperaturplateau exakt zu treffen, unter dem Risiko, die Hülle des Ballonkörpers zu überhitzen (over-shoot), oder durch mangelhafte Erhitzung nur unvollständig zu entspannen bzw. auszuformen (under-shoot).

Die "ballistische Durchwärmung" solcher Formteile von außen, also vom größten Durchmesser' des Ballonkörpers, nach innen hin, also zum in die Form eingebrachten Extrudat oder Rohschlauch, ist in der Regel auch insofern problematisch, als die äußeren Bereiche des Formwerkzeuges in zeitlicher Abfolge bzw. dem Energiefluß folgend, zuerst geheizt oder gekühlt werden, während die zentralen Anteile der Form, welche den auszuformenden Schlauchkörper aufnehmen und mit diesem formenden Kontakt haben, erst mit deutlicher zeitlicher Verzögerung auf das jeweils zu erreichende Niveau temperiert werden können.

Hierdurch bedingt können sich innerhalb der Hülle des Ballonkörpers unterschiedliche Entlastungsgrade bzw. Spannungszustände einstellen, die während des Blasvorganges zum Zerreißen des Ballons führen. Solche Spannungen treten zum Beispiel am Übergang des Rohschlauches in das eigentliche Ballonsegment, also im Bereich des unteren Ballonradius auf, da hier in der Regel die Wandstärke der Hohlkörperhülle am größten bzw. der auszuformende Schulterradius am kleinsten ist. Während die äußeren Hüllenanteile bereits entspannt sind, kann im rohschlauchnahen Teil des Werkzeuges bzw des Hohlkörpers noch hohe Spannung herrschen, und so das Reißen der Körperhülle verursachen.

Dies spielt insbesondere wiederum eine Rolle bei sehr großen Ballons, die von einem Rohschlauch mit kleinem Durchmesser ausgehend geformt werden, sowie bei Hohlkörpern mit aufwendiger, irregulärer Geometrie (z.B. nicht rotationssymmetrische Körper, tief und aufwendig taillierte Körper).

Um eine Über- oder Untertemperierung der für die erfolgreiche Ausformung spannungs-kritischen Portionen des Ballon- oder Hohlkörpers zu vermeiden, ist es bei der herkömmlichen Technik erforderlich, an den jeweiligen kritischen Massepunkten der Form Meßfühler zu platzieren, fortwährend Messwerte zu erfassen und der Steuerung der Temperierung zur Grundlage zu machen. Nur so kann die Reproduzierbarkeit des durch einen Temperaturgradienten angetriebenen Wärmeflusses zur formenden Werkzeuginnenfläche hin bzw. von dieser weg in einem großen oder komplex gestalteten Formwerkzeug gesichert werden.

Zwar ist bei kleiner dimensionierten und geometrisch einfachen Ballon- oder Hohlkörperformen die Platzierung einer ausreichenden Zahl punktuell messender Fühler in der Regel möglich und im Formenbau auch entsprechend integrierbar, bei größeren oder komplex gestalteten Körpern ist die Erreichbarkeit der jeweiligen formungs-kritischen Massepunkte durch den konzeptionellen Aufbau der Temperierungseinheit in der Regel entweder schwierig oder unmöglich.

Das ermittelte Temperaturmuster innerhalb der Form bildet selbst bei Verwendung vieler Messfühler lediglich einen punktuellen Ausschnitt der tatsächlichen, aktuell auf den zu formenden Körper wirkenden Formungstemperaturen ab, wobei zu bedenken ist, dass im Bereich eingesetzter Meßfühler die Struktur und damit das Temperaturverhalten erheblich anders ist als in den übrigen, nicht gemessenen Bereichen. Schließlich wird dadurch auch nicht die Temperatur an der Innenseite der Form gemessen, sondern allenfalls in deren Nähe. Die zeitsynchrone Darstellung der Temperierung sämtlicher Formsegmente im unmittelbaren Formungsbereich, also der Forminnenfläche, ist bei herkömmlichen Blasformeinheiten nach dem jetzigen Stand der Technik nicht möglich.

Derzeit verwendete Blasformungs-Maschinen (im folgenden auch als "Blower" bezeichnet), bestehen in der Mehrzahl aus einer äußeren Heiz- und Kühleinrichtung mit einer hohlzylindrischen Ausnehmung in der Mitte zur Aufnahme des eigentlichen Blasformwerkzeuges. Dieses hat einen dazu komplementären, zylindrischen Außenumfang, so dass es passgenau in die Ausnehmung der Heiz- und Kühleinrichtung eingesetzt werden kann. Die Blasformwerkzeuge haben eine innere Ausnehmung, deren Gestalt der gewünschten Ballonform entspricht; diese Gestalt ist in einen ansonsten massiven Block eingeschnitten bzw. eingedreht.

Blasformgeräte zur Fertigung medizinischer Ballon- oder Hohlkörperkomponenten, die dem aktuellen Stand der Technik entsprechen, werden überwiegend für die Fertigung von Ballons für die trans-luminale Aufdehnung von Gefäßen (Angioplastie) konstruiert und verwendet. Die verwendeten Ballon-Polymere sind üblicherweise Polyamid oder PET. Dieser spezifische, vor allem auch hochpreisige Anwendungsbereich gestattet eine wirtschaftliche Fertigung auch noch bei Zykluszeiten im Bereich mehrerer Minuten pro Ballon. Die Komponenten sind in der Regel klein dimensioniert, wiesen eine einfache symmetrische Geometrie auf und können bei entsprechend langen Durchwärmungsphasen des Formwerkzeuges relativ zuverlässig und gut reproduzierbar ausgeformt werden.

Eine entsprechende Blasformtechnologie wird beispielsweise von der Fa. Interface Associates, California, USA angeboten. Derartige Blower wurden überwiegend für die Fertigung von Ballon-Komponenten mit höchster mechanischer Belastbarkeit entwickelt. Dieser spezifische Anwendungsbereich erlaubt es bei der Blowergestaltung, die erforderlichen Leistungsmerkmale innerhalb bestimmter enger Grenzen zu halten. So liegen die erreichten Zykluszeiten im Bereich mehrerer Minuten. Es kann pro Fertigungszyklus in der Regel nur ein Ballon geformt werden.

Desweiteren sind für das Blasen hochwertiger, mechanisch beständiger Ballons glasform-basierte Techniken bekannt, wie z.B. von Farlow Glasblowing angeboten. Hier wird eine entsprechende Blasform aus Glas verwendet, die von außen mit einem Heißluftstrahl erhitzt und anschließend mit Pressluft abgekühlt wird. Problematisch ist dieses Verfahren in mehrfacher Hinsicht. Zwar erlaubt die Heißluft-Technik bei kleinen Ballons, wie sie für interventionelle intra-vaskuläre Anwendungen typisch sind kurze Zykluszeiten von unter einer Minute, sowie eine relative homogene Gleichverteilung der aufgenommenen Wärmeenergie in der Wandung der Glasform, für größere, oder komplex geformte Ballons ist das Verfahren jedoch nur bedingt tauglich. Wegen der mit steigendem Formdurchmesser ebenfalls steigenden Kraftentwicklung auf die Formflächen, können größere Ballons, die aus Materialien gefertigt sind, die hohe Blasdrucke zur Ausformung benötigen, nicht mehr gefahrlos in eine Glaskavität hineingeblasen werden. Ebenfalls sind bei vielen Materialien Trennmittel erforderlich, die die Entnahme der geblasenen Ballons ermöglichen bzw. erleichtern, jedoch für viele Anwendungsbereiche chemischtoxisch problematisch sind.

Angeboten werden darüber hinaus Ausformungstechniken, bei denen der Rohschlauch nach entsprechender partieller thermischer Konditionierung direkt in ein heißes flüssiges Medium eingebracht wird, und dort, quasi schwimmend auf eine durch die maximale spezifische radiale Expansion des Rohschlauches begrenzt in eine zylindrische Form gebracht wird. Einfache sphärische Formen bzw. komplexe Formen, oder eine gezielte Bestimmung des Dehnungsradius sind mit dieser Technik nicht möglich. Da in der Regel Wasser als Heizmedium verwendet wird, kann nur unterhalb des Siedepunktes ausformen, was bei vielen Materialien einen abschließenden zweiten separaten Dehnungsschritt erfordert.

Die Fertigung von Ballonkomponenten für den medizinischen Einsatz im oder am Patienten unterliegt zudem in der Regel besonderen hygienischen Bestimmungen. Höherwertige Katheterballons, beispielsweise für den intra-vas-kulären Einsatz, werden heute durch Maschinen gefertigt, welche ausnahmslos Desktop-Format aufweisen, und dabei derart konzipiert sind, dass alle zugehörigen Funktionseinheiten, wie vor allem die Temperierung, in der Fertigungseinheit selbst oder in deren unmittelbare Nähe zum Formungswerkzeug angebracht sind.

Kleinformatige Desktop-Blower, wie sie z.B-. von der Fa. Interface Associates, Laguna Niguel, California angeboten werden, sind für die Massenfertigung von höherwertigen medizinischen Ballonkomponenten nur bedingt geeignet. Zum einen sind die Geräte in der Regel derart ausgelegt, dass pro Formungszyklus nur ein einziger Ballon hergestellt werden kann, zum anderen ist auf der Grundlage dieser konventionellen Blasformtechnik die Ausformung großvolumiger Ballons bzw. aufwendig segmentierter Ballons mit komplexer Form technisch kaum möglich.

Um große Fertigungsstückzahlen zu erreichen, müssen entsprechend viele Blasformeinheiten mit jeweils zugehöriger Temperierungstechnik innerhalb eines reinen Fertigungsraumes bereitgestellt werden. Formungszeiten von in der Regel mehreren Minuten pro Stück schließen die wirtschaftliche Produktion höherer Stückzahlen, beispielsweise im Bereich mehrerer Hunderttausend oder Millionen pro Jahr, mit konventioneller Desktop-Technik für viele potentielle Ballonanwendungen aus.

Mit zunehmender Maschinenzahl bzw. zunehmender Anzahl von Temperierungseinheiten im Reinraum ergeben sich neben der hygienisch natürlich problemtischen hohen Anzahl von Maschinenbedienern auch von Seiten der einzelnen Fertigungsmaschinen her spezifische Probleme bei der Erhaltung des Reinraummilieus.

Beispielsweise basiert das Fertigungskonzept von Interface Associates auf einer lokalen Wasserkühlung mit einem Wasserreservoir, welches über ein jeweils separat angeschlossenes Kühlgerät temperiert wird. Sowohl das im Reinraum befindliche stationäre Wasser ist hinsichtlich seiner potentiellen bakteriellen Besiedelung hygienisch problematisch, als auch die permanente, vom Gebläse des Kühlaggerates erzeugte Verwirbelung von Luft in Bodennähe. Ferner kann sich auf den Oberflächen der Kühleinheit abtropfende Kondensation bilden, die ein anhaltend feuchtes Milieu unterhält. Die von den jeweiligen Kühleinheiten abgeführte Wärmeenergie wird zudem frei in den Reinraum abgegeben

Das alternativ beispielsweise von Farlow's Scientific Glasblowing, Grass Valley, California, angebotene Blasformverfahren basiert auf Heizung mit Heißluft, welche mit einem Gebläse als fokusierter Strahl auf das Formteil gerichtet wird. Die anschließende Abkühlung erfolgt mit einem entsprechenden Strahl sich entspannender Pressluft. Auch hier entstehen permanent Aufwirbelungen von Partikeln, die die Reinraumqualität beeinträchtigen können.

Bei permanenter Freisetzung von Wärmeenergie an vielen individuellen Produktionseinheiten innerhalb des Reinraums ist in der Regel eine zusätzliche Klimatisierung bzw. Temperaturregelung in der Reinraumtechnik erforderlich.

Besonders problematisch ist die Anwendung herkömmlicher Fertigungstechnologie bei großvolumigen oder komplex geformten Ballonkomponenten. Die erforderlichen Formteilmassen sind hier in der Regel so groß, dass die, primär für intra-vasale Ballons entwickelte Balsform-Technologie an die Grenzen ihrer Leistungsfähigkeit gerät und von den Desktop-Blowern maximale Heiz- und Kühlleistungen, mit entsprechender Beeinträchtigung der Reinraumqualität, abverlangt.

Ein weiteres Problem bei der Fertigung großer Stückzahlen auf einer Vielzahl individueller, sich autark versorgender Fertigungseinheiten, stellt die Prozesstabilität bzw. erreichbare Validierbarkeit des Prozesses dar. Jede einzelne Fertigungseinheit verfügt über eine Vielzahl zu messender und regelnder Funktionen. Der resultierende Validierungsaufwand ist erheblich, die Kontrolle der Prozesstabilität in laufender Fertigung entsprechend aufwendig.

Kommerziell verfügbare Blasformeinheiten sind zudem in der Regel nur für bestimmte Ballon-Polymere geeignet, während sich andere Grundstoffe, wegen der erforderlichen Formtemperatur oder Blasdrucke ausschließen.

Ein besonderer Nachteil bei herkömmlicher Formungstechnologie sind- nicht zuletzt die hohen Kosten, die durch den Erwerb der Fertigungseinheiten selber entstehen bzw. durch den laufenden Betrieb verursacht werden, da eine Vielzahl von Basisfunktionen, wie z.B. Energieversorgung, Druckluftaufbereitung, Steuerung, Dokumentation, Heizung und/oder Kühlung nicht zentralisiert, quasi redundant betrieben wird.

Ebenso sind bei konventioneller Technik die hohen Kosten der Formwerkzeuge sowie der den Werkzeugen angepassten Heiz- und Kühlummantelungen ein Faktor, der die rasche und kostengünstige Anpassung der Produktion an wechselnde Erfordernisse der Produktionskapazität erschwert.

Die EP 1 142 689 A offenbart ein Verfahren zur Herstellung von Blasformteilen aus kristallinem, thermoplastischen Harz, wobei geschmolzene Rohlinge in eine Form gebracht und dort geblasen werden, wobei das Blasen samt Formgebung etwa bei einer Temperatur um den Kristallisationspunkt des Harzes erfolgt, gefolgt von einem Abkühlen auf eine Temperatur unterhalb des Kristallisationspunktes, einer Haltephase bei dieser Temperatur und schließlich einer Zwangsabkühlphase. Dabei werden unterschiedlich temperierte Medien verwendet, um die Form aufzuheizen und abzukühlen. Zwar liegt der Hohlraum, durch den das Kühlmittel geleitet wird, etwa in der Mitte zwischen der inneren und äußeren Oberfläche der Form; die Temperierungsflüssigkeit wird jedoch durch Kanäle geleitet, welche zwischen dem Hohlraum für das Kühlmittel und der Innenseite der Form verlaufen.

Der US 5 993 721 A ist eine Form zur Verarbeitung eines geschmolzenen, thermoplastischen Harzes zu entnehmen. Ein Hohlraum zwischen der inneren und äußeren Oberfläche der Form erlaubt die Zirkulation eines Temperierungsmediums durch die Form; allerdings ist der Abstand dieses Hohlraums zu der äußeren Oberfläche der Form etwa fünf mal so groß wie der Abstand des Hohlraums zu der innern Formoberdläche.

Dasselbe gilt für die JP H04 351 526 A. Hier ist einerseits die äußere Gestalt der Formwandung völlig anders als die Gestalt der Forminnenwandung, und andererseits sind die Kanäle für die Zirkulation eines Temperierungsmediums in unmittelbarer Nähe zur Forminnenwandung geführt und gegenüber der Formaußenwandung durch Unterdruck führende Luftkammern thermisch isoliert.

In der US 2 222 461 A ist eine Form und ein Verfahren zur Herstellung von Strumpfformen aus Zelluloid. Die dabei verwendete Form wird aus einer Innenform und einer Außenform zusammengesetzt, zwischen denen ein Hohlraum verbleibt, worin ein Temperierungsmittel, insbesondere Dampf oder Wasser, zirkulieren kann. Allerdings haben beide zusammengesetzten Formen einschließlich des dazwischen liegenden Hohlraums eine Gesamtstärke von mindestens 3 cm, teilweise sogar mehr als doppelt so dick.

Aus diesen Nachteilen des beschriebenen Standes der Technik resultiert das die Erfindung initiierende Problem, eine gattungsgemäße Vorrichtung bzw. Verfahren derart weiterzubilden, dass die Temperaturentwicklung an der Grenzfläche zwischen Formwerkzeug und Hülle des auszuformenden Körpers einfach und allseitig beobachtet werden kann, wobei vorzugsweise hochwertige Ballonkomponenten in industriellen Fertigungsstückzahlen - insbesondere unter reinraumkompatiblen Bedingungen - auf ökonomische Weise hergestellt werden können. Darüber hinaus kann es von Vorteil sein, wenn sich zusätzlich noch eines oder mehrere der folgenden Ziele erreichen ließe(n):

- dass die Formwerkzeuge bzw. die ausformenden Einheiten von Blasformgeräten in ihrem Aufbau einfacher als bisher gestaltet werden können;
- das die Zahl der qualitätsrelevanten Prozessparameter durch den vereinfachten Aufbau des Formwerkzeuges reduziert und innerhalb enger Grenzen gehalten werden kann;
- dass durch die zeitsynchrone, fortlaufende Erfassung der Temperatur an der physischen Grenzfläche zwischen Ballon und Werkzeug auch großvolumige bzw. komplex gestaltete Ballons erfolgreich geblasen werden können;
- dass an den ausformenden Oberflächen des Werkzeugs eine zeitlich sequenzierte, segmentorientierte Temperierungsentwicklung erreicht werden kann;
- dass die Zykluszeit minimiert und die Stückzahl pro Herstellungszyklus maximiert wird;
- dass die bestmöglich validierbare industrielle Massenfertigung hochwertiger Ballonkomponenten ermöglicht wird;
- dass die Kosten für die Herstellung und Wartung von Formwerkzeugen reduziert werden können.
- dass die Ausformung auch großvolumiger oder komplex geformter Ballons, mit entsprechend hohen Formteilmassen, auf wirtschaftliche Weise unter Reinraumbedingungen möglich ist;

- das die Fertigungsparameter kontinuierlich, innerhalb sehr enger Grenzen gehalten werden können;
- dass die bestmögliche Validierbarkeit und In-Prozess-Kontrolle der Massenfertigung ermöglicht wird;
- dass die Mehrzahl der gängigen, für die Herstellung medizinischer Ballonkomponenten verwendeten Polymere mit der in der Erfindung vorgestellten Vorrichtung bzw. Anlage verarbeitet werden kann;
- dass die Anpassung des Prozesses an wechselnde Produktionskapazitäten rasch und kostengünstig erfolgen kann.

Weitere, spezifische Erfordernisse ergeben sich ggf. bei bestimmten Anwendungen. Bspw. sind Vorrichtungen zur kontinuierlichen Drainage von Stuhl aus dem Rektum eines Patienten in ein externes, beutelartiges Auffanggefäß seit einigen Jahren bekannt, in der klinischen Praxis jedoch bislang nur wenig etabliert.

Zur Ableitung bzw. zum Auffangen von Stuhl werden bei immobilen, nicht kooperativen Patienten überwiegend so genannte Fäkalkollektoren verwendet. Diese stellen beutelartige Strukturen dar, die in der Analfalte direkt über der Analöffnung aufgeklebt werden. Zwar ist die prä-anale Verklebung in der Regel über kürzere Anwendungsintervalle ausreichend haftend und dichtend, wegen des anhaltend feuchten und chemisch aggressiven Milieus im Verklebungsbereich sind jedoch häufig Mazerationen der exponierten Hautpartien zu beobachten.

Alternativ sind so genannte Darmrohre in Gebrauch, die über den Anus in den Enddarm eingeführt werden. Wegen des einhergehenden intra-rektalen Verletzungsrisikos sowie der permanenten Dilatation und damit möglichen Schädigung des analen Schließmuskels werden Darmrohre in der Regel nur zurückhaltend und passager zur Stuhlableitung verwendet.

Systeme zur weitgehend atraumatischen, langfristig kontinuierlich anwendbaren Stuhlableitung (indwelling fecal drainage), wie sie kürzlich bespielsweise von den Firmen Zassi Medical Evolutions Inc., Florida, USA und ConvaTec, New Jersey, USA, vorgestellt wurden, entsprechen in ihrem Aufbau konzeptionell den seit langem bekannten Blasenkathetern zur kontinuierlichen Drainage von Urin. Die bekannten Stuhldrainage-Systeme verfügen entsprechend über einen ballontragenden, die Drainage intra-rektal verankernden Kopfteil, sowie über ein sich daran anschließendes transanal, zu einem Sammelbeutel hin abführendes Schlauchelement.

Bei momentan verfügbaren kontinuierlichen Stuhldrainagesystemen wird nahezu ausnahmslos sowohl der intra-rektale Ankerballon, der sich anschließende trans-anale Schlauchanteil als auch der den Stuhl weiter zum Sammelgefäß hin abführende Schlauchanteil aus Silikon gefertigt. Bei angestrebter längerfristiger Verweilzeit von bis zu vier Wochen im Rektum eines Patienten ist Silikon jedoch in zweierlei Hinsicht besonders problematisch. Zum einen können druckbedingte Verletzungen im Bereich des terminalen Rektums (Druck-Ulcera) entstehen. Ebenfalls können mechanisch verursachte Läsionen im Bereich des Anus (Fissuren) beobachtet werden. Druck-Ulcera sind in der Regel die Folge unter Druck prall gefüllter, die Vorrichtung im Rektum verankernder Ballonkörper, welche den rektalen Strukturen dauerhaft anliegen und deren Durchblutung kritisch einschränken. Die im Markt bekannten Systeme besitzen ausnahmslos Silikon-Ballons, die im nicht befüllten Zustand in der Regel nur partiell ausgeformt sind, und durch Beaufschlagung mit ca. 100 mbar Fülldruck auf ihr tatsächliches Arbeitsmaß aufgedehnt werden, um so ihre intra-rektale Ankerfunktion zu gewährleisten. Anale Fissuren hingegen resultieren in der Mehrzahl aus der fortwährenden Reibung, die der in der Regel kollabierte, in dicker Faltung liegende trans-anale Schlauchabschnitt der Vorrichtung im Anus verursacht. Ein weiterer wesentlicher Nachteil bei der Verwendung von Silikonen als Basismaterial der stuhl-aufnehmenden und drainierenden Komponenten der Vorrichtung sind überdies die Besiedlungseigenschaften der Komponentenoberflächen mit Bakterien und Pilzen. Vor allem bei der Gabe von stuhlgängigen Antibiotika kann eine nahezu typische, reinigungsresistente mikrobielle Flora der Silikonoberfläche beobachtet werden, die ebenfalls antibiotische Resistenzen aufweisen kann, und bei vorhandenen Gewebeläsionen im Bereich des Anus und Rektum zu gefährlichen septischen Streuungen der auf den Oberflächen gedeihenden Keime führen kann. Die beschriebenebakterielle Überwucherung der Silikonoberflächen kann mit erheblicher Geruchsfreisetzung durch die Schlauchwandung hindurch, vor allem über den stuhlableitenden Zwischenschlauch zum Sammelbeutel hin, einhergehen. Bislang verfügbare Systeme aus Silikon wiesen zudem einen relativ komplexen Aufbau auf, und werden aus einer Vielzahl von Komponenten, in kostenintensiver manueller Arbeit verklebt. Silikon ist zudem, im Vergleich zu anderen gängigen Polymeren im Katheterbau, ein teurer Rohstoff. Ein besonderes, der Erfindung zugrunde liegendes Problem besteht daher darin, ein Verfahren und eine Vorrichtung zur Herstellung dünnwandiger Schlauchelemente vorzustellen, welche die oben beschriebenen Nachteile vermeiden, wobei die damit anzufertigenden Schlauchelemente insbesondere für die beschriebenen geruchs-hygienischen und infektions-relevanten Anwendungen bei silikonbasierten Systemen zur kontinuierlichen Stuhldrainage geeignet sein sollen.

Zur Lösung der obigen Probleme schlägt die Erfindung eine Vorrichtung gemäß Anspruch 1 und ein Verfahren gemäß Anspruch 10 vor.

Besonders vorteilhaft ist ein Aufbau des Formwerkzeuges, der auch in seinen Außenkonturen möglichst allseitig der Positivform des zu formenden Körpers entspricht, und dabei einen etwa kongruenten bzw. symmetrischen Aufbau der inneren und äußeren Formwandung aufweist. Die Symmetrie des Wandungsaufbaus im Verbund mit der Wiedergabe der Forminnenkontur auf der Formaußenwandung ermöglicht es, die wirksamen Formungstemperaturen an der Forminnenwandung, quasi indirekt, auf der gegenüberliegenden Fläche der Außenwandung in guter Näherung an den an der Innenseite wirkenden Wert zu erfassen.

Der strukturelle Aufbau und die Abmessungen der nach innen gerichteten Wandung der Form sind ihrer nach außen gerichteten Wandung so weit wie möglich kongruent bzw. konzentrisch. Dadurch haben die Innenfläche und die Außenfläche vorzugsweise an allen Orten etwa gleiche Abstände.

Durch den symmetrischen Wandaufbau des Formwerkzeuges entspricht die Temperaturentwicklung auf dessen Außenseite der Temperaturentwicklung auf dessen Innenseite nahezu exakt, so dass eine Abschätzung des Temperaturverlaufs, wie er sich an der Forminnenfläche darstellt, durch eine Ablesung des Temperaturverlaufs auf der Formaußenfläche in nahezu exakter Weise möglich ist. Durch eine Beobachtung der äußeren Formoberfläche kann daher die unmittelbare Formungstemperatur an der Innenseite der Form, wie sie auf den zu formenden Kunststoff einwirkt, in quasi indirekter Weise, dargestellt und im Verlauf beobachtet werden, z.B. durch Infrarot-Bildgebung über alle Flächen der Formaußenseite simultan, insbesondere in Echtzeit.

Beispielsweise können auf der Außenwandung leicht eine Vielzahl von Thermosensoren durch Klebung oder Pressung angebracht werden.

Ebenso gestattet der symmetrische Aufbau der Formwandung eine Beurteilung der tatsächlich wirkenden Formungstemperatur im direkten Übergangsbereich zwischen Forminnenwandung und Kunststoff durch Verwendung einer Wärmebildkamera. Die dort wirkende Energie stellt sich auf der Oberfläche des Werkzeuges entsprechend dar und kann durch eine Infrarotbildgebung kontinuierlich verfolgt und auf Synchronizität oder eine bestimmte gewünschte Temperierungssequenz der einzelnen Segmente des Werkzeuges hin überprüft werden. Ein Wärmebild bzw. - film der Formaußenwandung kann auch im laufenden Produktionsprozess leicht gemacht werden, Abweichungen im Wärmebild können einfach erkannt werden und bieten so eine optimale Grundlage für die Validierung des Formungsprozesses. Entscheidend beim Blasformen vor allem groß-volumiger, komplex gestalteter oder dünnwandiger Ballonfolien bzw. Hohlkörper ist eine möglichst homogene, exakt kontrollierbare zeitsynchrone Erwärmung sämtlicher Segmente bzw. formenden Oberflächen des verwendeten Formwerkzeuges.

Um dies zu erreichen geht die vorliegende Erfindung von der Verwendung vorzugsweise flüssiger Temperierungsmedien mit hoher Wärmetransportkapazität aus, und verzichtet bewusst auf andere Temperierungstechniken, wie z.B. durch elektrische Heizkomponenten wie Heizstäbe oder -wendeln, die bauartbedingt in der Regel keine verlässlich reproduzierbare Temperaturentwicklung sowohl im laufenden Betrieb der individuellen Einheit als auch beim Wechsel des Heizelementes erlauben. Ebenso bietet die Erwärmung des Formwerkzeuges mit Heißluft und Kühlung mit Pressluft keine Grundlage für einen verlässlich validierbaren bzw. reproduzierbaren Prozess.

Flüssige Temperierungsmedien können dem Formwerkzeug ohne großen technischen Aufwand über eine Rohrleitung zugeführt werden und innerhalb des Werkzeuges gezielt und gut dosierbar an formungskritische Punkte der Werkzeug-Innenfläche herangeführt werden. Die Temperierung des flüssigen Mediums bzw. der flüssigen Medien erfolgt, zur weitestmöglichen Reduktion von Meß- und Regelaufwand, vorzugsweise in einer zentralen Einheit. Die jeweiligen Medien werden vorzugsweise von dort durch eine Pumpfunktion permanent im Umlauf gehalten. Die einzelnen Fertigungseinheiten sind an diesen Temperierungskreisen angeschlossen.

Das Formwerkzeug kann neben einem heizenden und einem kühlenden Medium, wenn erforderlich, mit weiteren temperierenden Medien auf jeweilig präzise eingestellten Temperaturniveaus versorgt bzw. perfundiert werden.

Die Offenbarung schlägt als temperierendes Medium für alle verwendeten Temperaturniveaus vorzugsweise Wasser vor.

Da zur Verarbeitung verschiedener, momentan gebräuchlicher Polymere eine Temperierung des Mediums auf mindestens 200 Grad Celsius erforderlich ist, muss bei der Verwendung von Wasser in der flüssigen Phase im versorgenden System, sowie im Formwerkzeug selbst, ein dauerhafter Überdruck von ca. 20 bis 25 bar herrschen.

Werden innerhalb eines Fertigungszyklus mehrere Temperierungsniveaus eines Mediums verwendet, muss beim Wechsel sichergestellt sein, dass die jeweilig herrschenden Drucke im versorgenden System bzw. in der Form selbst soweit identisch sind, dass abrupte Ausdehnungen beim Wechsel eines Mediums zum anderen vermieden werden.

Um trotz der hohen Perfusionsdrucke und möglicher auftretender Entspannungseffekte des Mediums im Inneren der in der Werkzeugwandung liegenden flüssigkeitsführenden Kanäle die zu temperierende Formteilmasse so gering wie möglich zu halten, im Sinne einer möglichst raschen Temperierung die einen möglichst geringen oder keinen Temperaturgardienten zum Antrieb des Wärmeflusses erfordert, schlägt die Erfindung bevorzugt eine netzartig, zwischen den nach außen und innen begrenzenden Wandungen der Form angelegte und diese miteinander verstrebende Struktur kommunizierender, medium-durchströmter Räume vor.

Ein derartiges Perfusionsnetz besteht beispielsweise aus zuleitenden Kanälen, die über die Außenwandung an kritische Formpunkte, -flächen oder Segmente herangeführt werden, und endständigen, zu einer ableitenden Kanalstruktur überbrückenden, weitgehend flächig in der Formwandung temperierenden Hohlräumen. Flächig gestaltete Bereiche des Perfusionsraumes können durch räumlich komplexe, trabekelartige, wabenartige oder schwammartige Verstrebungen zwischen den Wandungen des Formteils bei geringst möglicher Aufwendung von Formmasse im sogenannten Laser-Cusing Verfahren hergestellt werden. Dabei wird Metallstaub in Lagen von wenigen Mikrometern Lagen flächig als quasi transversale Schichtung aufgetragen und anschließend durch einen Laserstrahl, einem Schnittbild durch das Werkzeug entsprechend, verschweißt bzw. gesintert. Laser Cusing erlaubt es, bei maximaler Materialersparnis, komplexe und stabile Kanalstrukturen aufzubauen, die mit anderen Formbauverfahren nicht oder nur mit größtem Aufwand hergestellt werden können.

So kann der das Medium führende, komplex verstrebte Raum nicht nur einem hohen Perfusionsdruck widerstehen, sondern auch dem vom Forminneren her wirkenden Blasformdruck im Werkstück. Selbst Formwerkzeuge mit einem Durchmesser von bis zu 100 mm und angewendeten Blasformungsdrucken von bis zu 3 bar können so sicher betrieben werden.

Die erreichbare Homogenität und Synchronizität bei der Erwärmung/Kühlung der erfindungsgemäßen masseleichten Form mit einem symmetrischem Wandaufbau kann eine nahezu zeitgleiche Entspannung aller Ballon/Hohlkörperteile erreicht werden. Asynchrone oder ungünstiger Spannungszustände innerhalb der Ballonhülle, die das Zerreißen eines Ballons begünstigen können vermieden, und auch kompliziert geformte Körper mit vielen, bei Aufdehnen vom Rohschlauch enstehenden Stresspunkten erfolgreich ausgeformt werden.

Es ist jedoch auch denkbar, durch eine entsprechende, den Fluß innerhalb der Kanäle modifizierende Gestaltung der Kanalführung so zu beeinflussen, dass sich eine zeitlich abgestufte Wärmeentwicklung im Formwerkzeug einstellt.

Die medium-führenden Kanälchen werden dabei optional derart angeordnet, dass für die Ausformung kritische Ballonsegmente mit punktuell erhöhter oder auch verminderter Intensität erwärmt werden können. So kann für die Entwicklung kritischer Ballonformen ein für die Ausformung erforderlicher zeitlicher Versatz bei der Erwärmung oder Kühlung bestimmter Formsegmente bis zum Erreichen einer homogenen Temperierung erreicht werden.

Die masse-leichte Ausführung der vorgeschlagenen Formwerkzeuge, bzw. das Verhältnis der Wärmekapazitäten zwischen dem Füllvolumen des Mediums und der Hohlform soll dabei vorzugsweise so gewählt werden, dass sich Erwärmung und Kühlung auf jeweils vorzugsweise 5 bis 30 Sekunden verkürzen.

Im idealen Fall wird das Verhältnis von Formteilmasse und der Masse des perfundierenden Mediums bzw. das Verhältnis der jeweiligen Wärmekapazitäten so gewählt, dass durch einmalige Injektion eines bestimmten Volumens von Medium die Temperatur an der Formwerkzeug-Innenfläche der Temperatur des Mediums selbst mit einer Abweichung von nicht mehr als 5 bis 10 % entspricht.

Die für den Blasformprozess erforderlichen Medien werden dem Formwerkzeug vorzugsweise über permanent zirkulierende, individuell geregelte Versorgungskreisläufe zugeführt und je nach Bedarf durch Schaltung vorgeordneter Ventile in das Formwerkzeug eingespeist.

Die Offenbarung bevorzugt ein Formwerkzeug mit einer überall möglichst gleichen Wandstärke, d.h., einem etwa konstanten Abstand zwischen innerer und äußerer, begrenzender Oberfläche.

Das Formwerkzeug kann einen möglichst dünnwandigen, masseleichten Aufbau der Formwandung aufweisen, bspw. mit einer Wandstärke von nicht mehr als 15 mm, vorzugsweise mit einer Wandstärke von nicht mehr als 12 mm, insbesondere mit einer Wandstärke von nicht mehr als 10 mm.

Hohlräume innerhalb der Formwandung zwischen der äußeren und inneren Wandung führen ein vorzugsweise flüssiges Temperierungsmedium.

Bevorzugt entspricht die Gestalt der äußeren Begrenzungsfläche der Wandung der inneren Begrenzungsfläche der Wandung, so dass diese Begrenzungsflächen etwa kongruent zueinander sind.

Durch eine nahezu perfekte Symmetrie kann dabei erreicht werden, dass sich die Temperatur an der Innenseite, d.h. an der Formungsoberfläche, von der Temperatur der Außenwand des Formwerkzeugs um weniger als 5 °C unterscheidet, vorzugsweise um weniger als 3 °C, insbesondere um 1 °C oder weniger.

Eine Maßnahme zur Erzielung möglichst perfekter Symmetrie besteht darin, dass das Formwerkzeug aus zwei zueinander konzentrischen, voneinander beabstandeten Flächen gebildet ist, deren einander zugewandte Innenflächen einen etwa konstanten Abstand voneinander aufweisen und einen geschlossenen Hohlraum begrenzen.

Die zwei zueinander konzentrischen, voneinander beabstandeten Flächen sollten jeweils eine gleiche Stärke aufweisen, bspw. je eine Stärke von 0,5 bis 8 mm, vorzugsweise jeweils eine Stärke von 1 bis 5 mm, insbesondere jeweils eine Stärke von 1,5 bis 3 mm.

Ferner sieht die Offenbarung vor, dass die zwei zueinander konzentrische, voneinander beabstandete Flächen ein- oder mehrmals miteinander verbunden sind, insbesondere durch ein oder mehrere stiftförmige Elemente. Diese Stifte haben mehrere Aufgaben: Einerseits gewährleisten sie, dass der Abstand zwischen äußerer und innerer Schale des erfindungsgemäßen Formwerkzeugs exakt konstant ist. Andererseits schaffen sie einen zusätzlichen, thermischen Kurzschluß zwischen beiden Schalen, so dass die Innen- und die Außentemperatur sich kaum voneinander entfernen können. Besonders wichtig ist aber ihre Wirkung auf das den Hohlraum durchströmende Medium: Für dieses bilden sie Strömungshindernisse, welche für eine turbulente Strömung in dem eingeschlossenen Hohlraum sorgen, so dass sich eine unregelmäßige Strömung einstellt, die im zeitlichen Mittel dazu führt, dass alle Teile des Formwerkzeugs gleichmäßig temperiert werden.

Indem die Längsachsen der stiftförmigen Elemente sich etwa lotrecht zu dem jeweils angrenzenden Bereich der beiden zueinander konzentrischen, voneinander beabstandeten Flächen erstrecken, ergibt sich eine maximale, mechanische Stabilität.

Indem einige, mehrere oder alle stiftförmigen Elemente in ihrer Längsrichtung von einem Entlüftungskanal durchsetzt sind, besteht die Möglichkeit, insbesondere an besonders kritischen Stellen einer Form, bspw. im Bereich einer Schulter, verdrängte Luft nach außen abzuleiten.

Die Erfindung läßt sich weiterbilden durch ringförmige Ein- oder Ausströmkanäle, welche über mehrere, entlang des Umfangs verteilte Öffnungen mit dem/den Hohlräumen innerhalb der Wandung kommunizieren. Diese ringförmigen Kanäle umschließen die Form entlang einer von der Längsachse etwa lotrecht durchsetzten Ebene und haben die Aufgabe, eine gleichmäßige Verteilung des strömenden Mediums und damit der Temperatur sicherzustellen.

Zwei oder mehrere, derartige, ringförmige Kanäle können miteinander verbunden und an einen gemeinsamen Ein- oder Auslaß angeschlossen sein. Dadurch läßt sich ein Medium in verschiedenen Ebenen entlang der Längsachse der Form zuführen bzw. ableiten, wodurch eine effektivere Temperierung erreicht wird.

Indem zwischen zwei miteinander verbundenen, ringförmigen Kanälen, welche an einen gemeinsamen Ein- oder Auslaß angeschlossen sind, wenigstens ein ringförmiger Kanal angeordnet ist, der als Auslaß bzw. Einlaß dient, ergibt sich allenfalls eine sanfte Temperaturvariation in axialer Richtung des Formwerkzeugs, so dass thermische Spannungen in dem Formwerkzeug vernachlässigt werden können.

Bevorzugt befindet sich wenigstens ein Einströmkanal in einem radial erweiterten Bereich der Form, während sich wenigstens ein Ausströmkanal in einem radial verjüngten Bereich der Form befindet. Damit findet die Temperierung in den radial erweiterten Bereichen statt; die radial verjüngten Bereiche des Formwerkzeugs können verzögert folgen, da die dortige Verformung minimal ist und daher weniger der thermischen Fixierung bedarf.

Das Formwerkzeug kann im so genannten Laser-Cusing-Verfahren hergestellt wird, einem Verfahren der dreidimensionalen, geschichteten Laser-Sinterung, wobei es Schicht für Schicht aufgebaut wird und so einen besonders masseleichten, hochdruck-beständigen Aufbau ermöglicht.

Durch das Herstellungsverfahren der dreidimensionalen, geschichteten Laser-Sinterung lassen sich räumlich komplexe Netze von mediumführenden Kanälen in die Formwandung integrieren.

Die mediumführenden Kanäle können auch bei geometrisch komplizierten oder mehrfach gekammerten Formen so entworfen und geführt werden, dass alle Formanteile bzw. deren Segmente zeitlich synchron oder in einer bestimmten zeitlichen Abfolge kontrolliert temperiert werden können.

Der Durchmesser einer erfindungsgemäßen Form kann - insbesondere an deren Innenseite - 50 mm oder mehr betragen, vorzugsweise 60 mm oder mehr, insbesondere 80 mm oder mehr.

Die Formwerkzeuge lassen sich mit einem großem Durchmesser von mehr als 50 mm bis zu 100 mm herstellen und erlauben einen sicheren Betrieb selbst bei der Anwendung von Blasformungsdrucken von bis zu 3 bar.

Andererseits widersteht ein erfindungsgemäßes Formwerkzeug trotz der niedrigen Formmassen Druckwerten der zu temperierenden Medien von bis zu 25 bar.

Durch die geringen verwendeten Außen- und Innenwandstärken des Formwerkzeuges im Verbund mit einer netz- bzw. stegartigen Verstrebung der gegenüberliegenden Formwandungen, kann die Masse bzw. die Wärmekapazität der Gesamtform deutlich reduziert werden, vorzugsweise auf Werte unterhalb der Wärmekapazität des Gesamtvolumens des in den Gesamthohlraum einfüllbaren Mediums, so dass sich bei der Übertragung von Wärmeenergie auf die Innenwandung der Hohlform die Temperatur des eingefüllten Mediums kaum ändert bzw. das Zeitintervall bis zur Angleichung der Temperatur der Forminnenwand und dem energiezuführenden oder-abführenden Medium sich auf wenige Sekunden verkürzt.

Ein Formwerkzeug zeichnet sich ferner dadurch aus, dass die Wandung des Formwerkzeuges in ihrem Aufbau derart beschaffen ist, dass der in die Werkzeugwandung integrierte medium-führende Innenraum nach außen und innen von einer Wandung jeweils identischer Stärke abgegrenzt ist, also einen symmetrischen Aufbau aufweist, der es erlaubt die Temperaturentwicklung auf der Innenseite (Formungsoberfläche) mit der Temperatur auf Außenwand des Formwerkzeuges gleichzusezten, und so punktuell oder flächig eine indirekte präzise Ablesung der Formungstemperatur an der Grenzfläche zum auszuformenden Produkt ermöglicht, wobei die derartige Wandungs-Symmetrie über die gesamte formungsrelevante Oberfläche des Formwerkzeuges ausgedehnt werden kann, oder auch nur als fensterartiger Ausschnitt in das Formwerkzeug integriert ist.

Anstelle der zuvor beschriebenen "ballistischen Temperierung" der Form über einen, den Energiefluß treibenden Temperaturgradienten wird die jeweils im Werkzeug zu erreichende Temperatur durch Fluten bzw. Perfundieren des medium-führenden Hohlraumes mit einem Medium definierter Temperatur erreicht - direkt, präzise, mit nur geringer zeitlicher Verzögerung sowie einem technisch und regeltechnisch insgesamt niedrigen Aufwand, wobei im Idealfall auf jegliche Temperaturfühlung im Werkzeug verzichtet werden könnte.

Insbesondere wenn wie die Offenbarung weiterhin vorsieht - In der Formwerkzeugwandung zwei oder mehrere, voneinander vollständig getrennte Kammern vorgesehen sind, können auch nicht-kompatible Medien bzw. verschiedene Medien räumlich getrennt voneinander geführt werden.

Die Offenbarung sieht den vorzugsweisen Betrieb des Werkzeuges in einer modular aufgebauten Fertigungsanlage vor, bei der der eigentliche Ort der Formung, die Blasformeinheit, von den rein versorgenden Funktionsanteilen wie Spannungsversorgung, Pneumatik-Versorgung, Temperierung und Prozess-Steuerung bzw. -statistische Dokumentation räumlich getrennt ist, und dieser bevorzugt außerhalb der eigentlichen Fertigungsumgebung bzw. der stationiert wird. Das Formwerkzeug kann an einen oder mehrere zentrale Versorgungsstränge angebunden werden, welche die zur Ausformung erforderlichen Medien auf entsprechendem Temperaturniveau bereitstellen.

Es liegt im Rahmen der Erfindung, dass das Formwerkzeug an einen oder mehrere zentrale Versorgungsstränge angebunden ist, die die zur Ausformung erforderlichen Medien auf entsprechendem Temperaturniveau bereitstellen.

Eine Vorrichtung zur möglichst partikel- und keimarmen Massenfertigung von Ballonkörpern in einer Reinraum-Produktionsumgebung, zeichnet sich ferner aus durch eine außerhalb der Reinraum-Produktionsumgebung angeordnete Einrichtung zur Temperierung eines Mediums sowie durch eine Einrichtung zur Zuführung des temperierten Mediums zu wenigstens einem in der Reinraum-Produktionsumgebung befindlichen Formwerkzeug.

Es hat sich als bewährt, dass die Einrichtung zur Temperierung eines Mediums als Heizeinrichtung ausgebildet ist. Die Erfindung läßt sich dahingehend weiterbilden, dass die Einrichtung zur Zuführung des temperierten Mediums zu wenigstens einem in der Reinraum-Produktionsumgebung befindlichen Formwerkzeug wenigstens eine Rohr- und/oder Schlauchleitung umfaßt.

Es hat sich als günstig erwiesen, dass in der Leitung für das temperierte Medium, vorzugweise außerhalb der Reinraüm-Produktionsumgebung, wenigstens eine Fördereinrichtung angeordnet ist, insbesondere eine Förderpumpe.

Weitere Vorteile ergeben sich daraus, dass die Leitung für das temperierte Medium als vorzugweise außerhalb der Reinraum-Produktionsumgebung in sich geschlossene Ringleitung ausgebildet ist, so dass eine ständige Zirkulation des Mediums möglich ist.

Bevorzugt ist außerhalb der Reinraum-Produktionsumgebung eine Steuer- und/oder Regeleinrichtung vorgesehen.

Die Temperierung des Formwerkzeuges kann ausschließlich durch flüssige Medien erfolgen.

Die Offenbarung empfiehlt, dass als Heiz- und Kühlmedium Wasser, vorzugsweise in der flüssigen Phase, verwendet wird.

Es liegt im Rahmen der Erfindung, dass in einer Leitung für ein Temperiermedium ein Druck von 10 bar oder mehr herrscht, vorzugsweise ein Druck von 15 bar oder mehr, insbesondere ein Druck von 20 bar oder mehr.

Eine weitere Konstruktionsvorschrift sieht vor, dass weitere oder sämtliche, die Qualität des Reinraumes potentiell beeinträchtigenden Funktionen außerhalb des Fertigungsbereiches angeordnet sind, vorzugsweise zusammengefasst in einer zentralen Versorgungseinheit.

Mit großem Vorteil erfolgen Energieversorgung, Aufbereitung und Transport aller erforderlichen Medien sowie insbesondere die Temperierung der Heiz- und Kühlmedien in einer zentralen Einheit mit zentraler Regeltechnik.

Es hat sich als günstig erwiesen, dass sich im Reinraum selbst nur eine oder mehrere Andock-Stationen befinden, an denen (je) eine entsprechend ausgelegte Fertigungsstation mit minimaler, die Qualität des Reinraum-Milieus nicht beeinträchtigender Komponentenausstattung, anschließbar ist.

Die Anschlüsse in dem Reinraum zur Ankopplung (je) einer Fertigungsstation können als selbsttätig schließende und öffnende Kupplungen ausgebildet sein, so dass der Formteilwechsel an einer Fertigungsstation rasch und komfortabel durchgeführt werden kann.

Die Erfindung bietet die Möglichkeit, dass an freie Docking-Stationen jederzeit zusätzliche Fertigungsstationen anschließbar sind.

Es liegt im Rahmen der Erfindung, dass Fertigungsstationen jederzeit an- oder abgeschlossen werden können, vorzugsweise ohne die Versorgungseinheit(en) ab- oder anzuschalten. '

Die Offenbarung sieht weiterhin vor, dass ein oder mehrere, vorzugsweise alle zentral bereitgestellten Medien, ein oder mehrere, vorzugsweise alle am Fertigungsplatz erfassten Informationen, und/oder ein oder mehrere, vorzugsweise alle Steuersignale für den Fertigungsplatz so weit wie möglich über Versorgungsstränge außerhalb des Reinraumes geführt und der dort platzierten Zentraleinheit zugeleitet sind.

Es hat sich als bewährt, dass mehrere Anschlüsse für je eine Fertigungsstation an einer sich vorzugsweise vom Boden bis zur Decke des Reinraums erstreckenden Säule angeordnet sind, insbesondere etwa ring- oder rosettenförmig um die Säule verteilt.

Die Erfindung läßt sich dahingehend weiterbilden, dass ein oder mehrere, vorzugsweise alle zentral bereitgestellten Medien, ein oder mehrere, vorzugsweise alle an den betreffenden Fertigungsplätzen erfassten Informationen, und/oder ein oder mehrere, vorzugsweise alle Steuersignale für die betreffenden Fertigungsplätze durch die betreffende Säule geführt ist/sind.

Jede Fertigungsstation kann über die Möglichkeit verfügen, in parallelen Strängen simultan mehrere Schlauchelemente aufzunehmen und zu Ballons auszuformen.

Die Offenbarung zeichnet sich weiterhin aus durch eine Schnellwechsel-AnschlußVorrichtung für ein Werkzeug, so dass sich durch den Werkzeugwechsel im Prozess keine bzw. nur minimale Parameterverschiebungen einstellen.

Die vorliegende Erfindung beschreibt ferner ein gattungsgemäßes Verfahren zur Herstellung von Ballon- oder Hohlkörpern aus einem schlauchförmigen Rohling durch Blasformen mit wenigstens einer temperierbaren Hohlform, innerhalb welcher der Rohling bis zu seiner gewünschten Gestalt aufgebläht wird, zeichnet sich dadurch aus, dass ein Hohlraum in der Wandung der Hohlform mit einem vorzugsweise flüssigen Medium befüllt wird. Dabei überträgt das eingefüllte Medium einen (kleinen) Teil seiner Wärmemenge auf die Hohlform, es findet innerhalb weniger Sekunden ein. Temperaturausgleich statt, Da nur eine vergleichsweise geringe Wärmemenge übertragen wird, um den sehr dünnwandigen Formkörper zu erwärmen bzw. zu kühlen, ist das thermische Gleichgewicht in einem sehr kurzen . Zeitraum erreicht, die Zykluszeit, welche maßgeblich durch die Zeitintervalle zum Aufwärmen bzw. vollständigen und gleichförmigen Durchwärmen der Hohlform von der Entnahme-Temperatur auf die Formungs-Temperatur einerseits und zum späteren Abkühlen zurück auf die Entnahme-Temperatur andererseits bestimmt wird, kann daher selbst bei großen Ballonformen (beispielsweise bis zu 10 cm messenden Durchmessern) und aufwendigen Ballongeometrien (beispielsweise Ballonformen mit Segmenten deutlich unterschiedlicher Segmentdurchmesser) auf einen Bruchteil der bisher dafür benötigten Zeit reduziert werden, bspw. auf Zeiten deutlich unterhalb einer Minute, so dass - in Verbindung mit einer Automatisierung der Beschickung und Entformung - eine bedeutend rationellere und damit wirtschaftlichere Herstellung möglich ist als bisher.

Der Hohlraum kann in zeitlichen Abständen, intermittierend mit demselben Medium gespült werden, um die Temperatur der Hohlform konstant zu halten.

Die Erfindung läßt sich dahingehend weiterbilden, dass der Formgebungsvorgang (Einführen eines Rohlings, Vorwärmen, Streckung, Aufblähen, Entspannung, Kühlung und Ausstoß) ausschließlich zeitgesteuert abläuft. Da - im Gegensatz zur konventionellen Technik - die Temperatur der Hohlform bereits nach einem sehr kurzen Zeitraum von einigen Sekunden im thermischen Gleichgewicht mit dem eingefüllten Medium steht und in sämtlichen Segmenten die gewünschte Temperatur angenommen hat. Auf eine Temperaturerfassung an der Hohlform samt übergeordneter Temperaturregelschleife - wie bisher notwendig - kann daher verzichtet werden.

Die Lösung des oben genannten Hauptproblems gelingt im Rahmen einer gattungsgemäßen Vorrichtung dadurch, dass die gesamte Temperierung des Formwerkzeuges durch Zuführung flüssiger Temperierungsmedien von einem zentral versorgenden, steuernden und regelnden Modul außerhalb des Reinraum-Fertigungsbereiches aus erfolgt.

Die Offenbarung sieht einen modularen Aufbau einer entsprechenden Fertigungsanlage vor, bei der die Funktionsanteile: Spannungsversorgung, Pneumatik-Versorgung, Temperierung und Prozess-Steuerung bzw. -statistische Dokumentation in einem Komplex zusammengefasst sind, und dieser bevorzugt außerhalb der eigentlichen Fertigungsumgebung stationiert wird.

Die eigentlichen Fertigungseinheiten sind eng auf ihre zu erfüllende Fertigungsleistung ausgelegt und werden über ein Ringleitungssystem mit den Temperierungsmedien bzw. allen anderen erforderlichen Energien und Medien versorgt. Auch die Steuerungslogik der einzelnen Fertigungsplätze befindet sich in der Zentraleinheit und kommuniziert von dort mit dem lokalen Messfühlern und ausführenden Elementen über ein Datenbuskabel. Über ein lokal vorhandenes Visualisierungs- und Eingabeportal kann über diesen Bus auf alle zentralen Funktionen zugegriffen werden.

Vor allem die Zentralisierung der Temperierung, welche Heizung, Kühlung, Pufferfunktion, Pumpfunktion und Regelungstechnik zusammenfasst, bedeutet einen deutlich vereinfachten Aufbau der eigentlichen Blasformeinheit, wodurch diese sehr kostengünstig vervielfältigt werden kann, was wiederum eine kostengünstige und rasche Kapazitätsanpassung bei der Produktion ermöglicht.

Damit lassen sich die Merkmale realisieren,

- dass die Temperierung des Formwerkzeuges ausschließlich durch flüssige Medien erfolgt;
- dass als Heiz- und Kühlmedium vorzugweise Wasser in der flüssigen Phase verwendet wird;
- dass sämtliche, die Qualität des Reinraumes potentiell beeinträchtigenden Funktionen außerhalb des Fertigungsbereiches in einer zentralen versorgenden Einheit zusammengefasst sind;
- dass Energieversorgung, Aufbereitung und Transport aller erforderlichen Medien sowie insbesondere die Temperierung der Heiz- und Kühlmedien in einer zentralen Einheit mit zentraler Regeltechnik erfolgt;
- dass sich im Reinraum selbst nur noch Andock-Stationen befinden, an denen entsprechend ausgelegte Fertigungsstationen mit minimaler, die Qualität des Reinraum-Milieus nicht beeinträchtigenden Komponentenausstattung, angeschlossen werden;
- dass zusätzliche Fertigungsstationen kostengünstig erworben, und eine Anpassung an die erforderliche Fertigungskapazität rasch und technisch unkompliziert erfolgen kann;
- dass alle zentral bereitgestellten Medien, alle am Fertigungsplatz erfassten Informationen, sowie alle Steuersignale für den Fertigungsplatz so weit wie möglich über Versorgungsstränge außerhalb des Reinraumes geführt und der dort platzierten Zentraleinheit zugeleitet werden;
- dass die Formwerkzeuge vorzugsweise im kostengünstigen Laser-Cusing Verfahren hergestellt werden;
- dass die Fertigungswerkzeuge so besonders masseleicht und optimal kleiner, zur Umgebung hin ernergie-emittierender Oberfläche ausführbar sind;
- dass Formwerkzeuge so auch für große Ballonkörper bzw. komplex geformte Komponenten masseleicht und kostengünstig hergestellt werden können;

- dass die so hergestellten Formwerkzeuge trotz der niedrigen Formmassen Druckwerten der temperierenden Medien von bis zu 25 bar widersteht;
- das auch Formteile mit maximalen Innendurchmesser bis zu 100 mm und angewendeten Blasdrucken von bis zu 3 bar sicher betrieben werden können
- und in modularer serieller Anordnung die sequentielle Ausformung von Ballons aus einem einzigen Rohschlauchelement erlaubt;
- dass jede Fertigungsstation über die Möglichkeit verfügt in parallelen Strängen simultan mehrere Schlauchelemente aufzunehmen und zu Ballons auszuformen;
- dass der Formteilwechsel an der Fertigungsstation rasch und komfortabel durchgeführt werden kann;
- dass sich durch den Werkzeugwechsel im Prozess keine bzw. nur minimale Parameterverschiebungen einstellen;
- das auch Formteile mit großem Durchmesser bis zu 100 mm und angewendeten Blasdrucken von bis zu 3 bar sicher betrieben werden können
- dass die zuvor beschriebene ballistische Temperierung über einen Temperaturgradienten entfällt, und die jeweils im Werkzeug zu erreichende Temperatur durch Fluten des Hohlkörpers mit einem Medium definierter Temperatur direkt, präzise, mit nur geringer zeitlicher Verzögerung sowie einem technisch und regeltechnisch insgesamt niedrigen Aufwand, erreicht werden kann, wobei im Idealfall der gänzliche Verzicht auf Temperaturfühlung im Werkzeug eine Option ist;
- das die Formteiltechnologie auch die Kombination nicht kompatibler Temperierungsmedien ermöglicht wird.

Flüssige Temperierungsmedien können dem Formwerkzeug dem jeweiligen Arbeitsplatz ohne großen technischen Aufwand über eine Rohrleitung zugeführt werden. Die Temperierung des Mediums selbst wird erfindungsgemäß zur weitestmöglichen Reduzierung von Heiz- und Regelungsaufwand in einer einzigen zentralen Einheit zusammengefasst.

Die Offenbarung schlägt als Temperierungsmedium insbesondere Wasser vor. Da zur Gewährleistung der Verarbeitung aller momentan gebräuchlichen Ballonmaterialien eine Temperierung des Mediums auf mindestens 200 Grad Celsius erforderlich ist, muss bei der Verwendung von Wasser in der flüssigen Phase im Gesamtsystem ein Überdruck von ca. 20 bis 25 bar dauerhaft herrschen.

Temperierungsmedien:

Die Vorrichtung verfügt über mindestens ein, zwei oder mehrere Temperierungskreisläufe. Es werden bevorzugt flüssige Medien oder Medien in der Dampfphase verwendet. Vorzugsweise wird für Heizung als auch Kühlung Wasser verwendet. Um einen Betrieb über Umgebungsdruck zu vermeiden kann die Heizung auch mit hochtemperatur-tauglichen Ölen oder anderen Flüssigkeiten mit ausreichend hohem Siedepunkt von mindestens 200 Grad Celsius erfolgen. Idealerweise werden für Temperierung und Kühlung identische Medien verwendet. Alternativ ist beispielsweise jedoch eine Erhitzung mit Ölen und Kühlung mit Wasser denkbar, was allerdings eine aufwendige strikte räumliche Trennung der beiden Funktionen im Formwerkzeug erfordert.

Temperierungskreisläufe:

In der einfachsten Ausführung verfügt das versorgende Kreissystem nur über ein einziges Temperierungsniveau, welches auf die eigentliche Formtemperatur eingeregelt ist. Die Kühlung kann durch ein lokal zugeführtes oder erzeugtes, und am Ort der Verwendung verworfenes Medium, wie z.B. Leitungswasser oder Pressluft erfolgen.

Vorteilhaft wird die Kühlung jedoch in Form eines flüssigen Mediums im Kreissystem, idealerweise als in sich geschlossenes System, welches nach extern über einen Wärmetauscher kommuniziert, bereitgestellt. Beispielsweise können in beiden Kreisläufen identische Thermoöle zirkulieren.

Beim Blasformen spezifischer Materialien können allerdings mehrere Temperierungsniveaus erforderlich sein. Beispielsweise erfordert die Blasformung von Polyurethan (PUR) zwei Temperaturniveaus, wobei die obere Temperatur der zur Ausformung und völligen Entspannung erforderlichen Temperatur entspricht, in der Regel bei Polyurethan etwa 160 bis 170 Grad Celsius, die untere Temperatur hingegen sowohl für die Entnahme des Ballons aus der Form geeignet ist als auch für die Vortemperierung des Schlauchrohlings verwendet werden kann. Diese Temperatur liegt etwa bei 40 bis 60 Grad.

Optional, sollte die Entnahme des Ballons bei Vorwärmtemperatur nicht möglich sein, kann ein eigener Kühlkreislauf zugeschaltet werden.

Sollte sich bei der Ausformung eines bestimmten Ballons die Notwendigkeit eines weiteren verfügbaren Temperaturniveaus ergeben, kann dies in entsprechender Weise als geregelter Kreislauf angeboten und von einer lokalen Ventilanordnung gesteuert in die Hohlform injiziert werden.

Die Offenbarung beschreibt beispielhaft eine Fertigungsanlage mit zwei Temperierungskreisen sowie einem Kühlkreis, wobei alle Kreise Wasser in der flüssigen Phase als Transportmedium verwenden.

Flüssiges Wasser weist eine vergleichsweise hohe Wärmetransportkapazität auf. Bei ca. 20 bar kann auf 200 Grad Celsius temperiertes Wasser in flüssiger Phase gehalten werden. Bevorzugt stellt ein Kreissystem flüssiges Wasser auf dem Niveau der Ausformung bereit, welches in der Regel im Bereich zwischen 100 und 200 Grad Celsius liegt.

Ein weiteres Niveau wird im Temperaturbereich zwischen 40 und 100 Grad Celsius bereitgestellt. Es entspricht einer Temperatur, die den Rohschlauch in der Phase vor der Beaufschlagung mit dem Blasdruck konditioniert.

Der Wechsel von einem Temperaturniveau über dem Siedepunkt auf ein Niveau unterhalb des Siedepunktes geht bei flüssigen Medien mit einer abrupten Ausdehnung des über Siedetemperatur erhitzten Mediums einher. Der Wechsel muss daher zu einem Medium unter gleichem Druck erfolgen. Die Vorrichtung enthält daher eine druckregulierende Komponente, die die Perfusionsdrucke in allen heizenden Kreisläufen auf gleichem Niveau hält. Temperierungssysteme, die über zwei entsprechend druckregulierte Heiz-Module auf Wasserbasis verfügen, werden z.B. voh der Fa. Single Temperierungstechnik hergestellt.

Bei Verwendung einer derartigen Heiztechnik, die es erlaubt, von einem hohen druck-beaufschlagten Niveau auf eine Temperatur unterhalb des Siedepunktes zu wechseln, erlaubt den problemlosen Wechsel auf Kühltemperatur-Niveau.

Die einzelnen Temperierungs-Heizer verfügen über Pumpen, welche das jeweilige Medium in einem Rohrleitungssystem kreisen lassen. Zwischen Vor- und Rücklauf ist ein druck- oder flußminderndes Element eingebaut welches den, einem so entstehenden Druckgradienten folgenden Überstrom des Mediums vom Vor- zum Rücklauf über das Formwerkzeug sicherstellt. Die Versorgungskreisläufe können weiterhin großvolumig ausgelegte Reservoirbehälter (z.B. 25 bis 50 Liter Füllvolumen) integrieren, um so Temperaturverschiebungen im Medium durch Wärmeaufnahme oder -abgabe aus dem Formwerkzeug bereits weitestgehend abzupuffern, und die simultane Versorgung mehrerer am Kreislauf angekoppelter Blasformstationen zu ermöglichen.

Die Sollwertregelung im jeweiligen Kreis orientiert sich an im Versorgungskreislauf angebrachten Istwert-Fühlern bzw. durch möglichst formwerkzeugnahe Fühler und ist für jeden Versorgungskreis autark.

Temperatursensing:

- über die Formaußenfläche (symmetrischer Aufbau der Formwandung, Wärmeentwicklung auf der Außenseite entspricht der der Innenseite, entweder mit gesteckten, geklemmten, aufgeklebten Fühlern, oder bildgebend, die Erwärmung der Gesamtform im Verlauf erfassend, mit IR-Kamera)
- über die Form-Innenwandung (die Wandung durchdringender Kanal, steckbarer Sensor vorgeschoben bis auf oder in die Forminnenwand)
- optionaler Verzicht auf Fühler, da rascher Angleich von Formtemperatur und Mediumtemperatur

Die Ankoppelung von Arbeitsstationen an die jeweilige Versorgungsleitung erfolgt durch Verschraubung und ist durch ein manuell betriebenes Sperrventil gesichert.

Die Offenbarung geht ferner davon aus, dass zur formkonstanten Ausformung von Ballons in der Grenzfläche zwischen Werkzeug und Ballonhülle bestimmte Temperierungsniveaus exakt reproduzierbar erreicht werden müssen. Da zur Verarbeitung einzelner Materialien innerhalb eines Fertigungszyklus mehrere Temperierungsniveaus, einstellbar sein müssen, muss beim Wechsel sichergestellt sein, dass die jeweilig herrschenden Drucke innerhalb der Versorgungsleitungen soweit identisch sind, dass abrupte Ausdehnungen des Mediums vermieden werden. Um trotz der herrschenden hohen Perfusionsdrucke im Inneren der im Werkzeug liegenden flüssigkeitsführenden Kanäle die zu temperierende Formteilmasse so gering wir möglich zu halten, schlägt die Erfindung bevorzugt eine netzartige Kanalstruktur vor, welche in die Wandung des Formwerkzeuges eingearbeitet ist.

Derartige komplexe Strukturen, bestehend aus beispielsweise zuleitenden Kanälen, diese miteinander verbindende Kanalstrukturen und endständigen, flächig temperierende, netzartige Hohlräumen, können unter bei geringst möglicher Aufwendung von Masse im sogenannten Laser-Cusing Verfahren, aus laserverschweißtem Metallstaub hergestellt werden. Metallstäube werden dabei in wenige Mikrometer messenden Schichten flächig aufgetragen und anschließend durch einen Laserstrahl, einem transversalen Schnittbild durch das Werkzeug entsprechend, verschweißt bzw. gesintert. So ist es möglich, maximal materialsparend, komplexe räumliche Strukturen aufzubauen, die mit anderen Formbauverfahren nicht oder nur mit größtem Aufwand hergestellt werden können.

Die Kanälchen der Temperierungs-Endstrecke können beispielsweise flächendeckende Netzwerke darstellen, wobei ein flächiger Hohlraum niedriger Höhe, von beispielsweise einem Millimeter, durch stegartige Strukturen oder Pfeiler welche in regelmäßigen Abständen Boden und Decke der Räume verbinden, in einem regelmäßigen Muster stabilisiert wird, und so dem hohen Perfusionsdruck standhält.

Die medium-führenden Kanälchen werden dabei optional derart über die Oberfläche der Formwerkzeuge-Innenfläche angeordnet, dass für die Ausformung kritische Ballonsegmente punktuell mit erhöhter oder auch verminderter Intensität erwärmt werden können. So kann für die Entwicklung kritischer Ballonformen ein für die Ausformung erforderlicher zeitlicher Versatz bis zur homogenen Durchwärmung erreicht werden.

Durch die erreichbare Homogenität und Synchronizität in der Erwärmung/Kühlung bzw. deren gezielte Beeinflussung durch Schaffung einer zeitlich abgestuften Wärmeentwicklung im Formwerkzeug, können sämtliche Segmente des auszuformenden Ballons besonders vorteilhaft, unter Vermeidung asynchroner oder ungünstiger Spannungszustände innerhalb der Ballonhülle, die das Zerreißen eines Ballons begünstigen können, erfolgreich ausgeformt werden.

Besonders vorteilhaft ist ein Aufbau des Formwerkzeuges, der in seinen Außenkonturen allseitig der Grundform der Positivform des zu formenden Körpers im Wesentlichen entspricht und, so weit wie möglich, gleiche Wandstärken der Form-Innen- und Form-Außenwand aufweist. Die so erreichte Symmetrie gestattet die Messung der Formtemperatur der Forminnenwandung auf der Außenwandung. So können auf der Außenwandung leicht entsprechende Thermosensoren angebracht werden. Ebenso gestattet der symmetrische Aufbau der Formwandung eine Beurteilung der tatsächlich wirkenden Formungstemperatur im direkten Übergangsbereich zwischen Forminnenwandung und Kunststoff durch Verwendung einer Wärmebildkamera. Die dort wirkende Energie stellt sich auf der Oberfläche des Werkzeuges entsprechend dar und kann durch eine Infrarotbildgebung kontinuierlich verfolgt und auf eine gewünschte Temperierungssequenz der einzelnen Segmente des Werkzeuges hin überprüft werden. Abweichungen im Wärmebild können einfach erkannt werden.

Alternativ zu der bevorzugten Gestaltung eines masse-leichten Werkzeuges, kann eine masse-schwere Form verwendet werden, bei der die temperierende Kanal- und Netzstruktur unmittelbar unterhalb der Formteil-Innenfläche eingebaut ist. Der nach außen hin gerichtete Werkzeuganteil kann im Grunde beliebig schwer sein. Ein derartiger Aufbau kommt vor allem dann in Frage, wenn die Abfolge der Blasformzyklen exakt getaktet ist bzw. die Zuführung und Entnahme von Rohschlauch und Ballon automatisiert ist. Bei nicht gewährleistetem Gleichtakt, sind, bedingt durch die Wärmespeicherkapazität der Schweren äußeren Hülle Schwankungen der Formtemperatur denkbar.

Die für den Blasformprozess erforderlichen Temperaturniveaus werden in Form zirkulierender, individuell regulierter Versorgungskreisläufe angeboten und je nach Bedarf durch Schaltung von Ventilen in das Hohlwerkzeug eingespeist.

Blasvorrichtung-Workstation

Die die Blasform aufnehmende eigentliche Fertigungsvorrichtung besteht bei der beschriebenen Ausführung im Wesentlichen aus einem Mechanismus zur beidseitigen Klemmung bzw. Inflation des Rohschlauches, einem Mechanismus zur axialen Streckung des Schlauches, einem vorzugsweise rückgekoppelten Regelkreis zur Steuerung des Blasdruckes sowie einem des Blasfluss regulierenden Stellglied.

Die Fertigungsvorrichtung wird direkt an die verschieden temperierten Versorgungskreisläufe angekoppelt, der Fluß des Temperierungsmediums wird durch ein Set strömungsregulierender Ventile geschaltet. Die Gesamtsteuerung der Einheit erfolgt über einen Klein-Steuerungsrechner, in den das jeweilige Fertigungsprogramm überspielt wird.

Die Regelung der verschiedenen Temperierungsmedien erfolgt rückgekoppelt durch separate Regelkreise durch Temperaturfühler, die vorzugsweise nahe den Ankopplungspunkten der Fertigungseinheiten platziert sind.

Für die Fertigung von Polyurethan-Ballons wäre zum Beispiel folgende Kombination von Medien typisch: Die Temperierung eines Vorratsbehälters kann der Entspannungs- bzw. Ausformungstemperatur entsprechen. Diese Temperatur liegt bspw. bei einer Temperatur zwischen 150 °C und 180 °C, vorzugsweise zwischen 160 °C und 170 °C.

Für die Kühlung auf Entnahmetemperatur des entspannten und geformten Ballons kann in der Regel die gleiche Temperierung wie für die Vorwärmung des Rohlings innerhalb der Hohlform verwendet werden. Dieser Wert liegt bspw. bei einer Temperatur zwischen 40 °C und 80 °C, vorzugsweise etwa zwischen 50 °C und 60 °C.

Sollte zur leichten Entformung bzw. Entnahme eines beispielsweise extrem dünnwandigen oder klebrigen Ballons zur separaten Kühlung eine von der Vorformungstemperatur unabhängige, niedrigere Temperatur erforderlich sein, zum Beispiel etwa 10 bis 20 Grad C, kann diese über einen entsprechend geregelten Kreislauf zur Verfügung gestellt werden. Auch dieses Kühlmedium unterliegt dem selben Druck wie die Kühlmedien für die anderen beiden Temperaturniveaus.

Ein Medium kann dazu verwendet werden, um das bisherige Medium aus dem Hohlraum an der Außenseite der Hohlform herauszudrücken, indem das einströmende Medium das vorige Medium in seinen zugehörigen Versorgungskreislauf verschiebt. Diese Separierung von Medien kann fix zeitgesteuert sein oder sich an gemessenen Temperaturen orientieren. Einer Temperatur-Nachregelung der Medien durch ungünstige Vermischung verschieden temperierter Medien kann so weitgehend vorgebeugt werden.

Serielle Fertigung:

Die Erfindung bietet die Möglichkeit, dass mehrere Ballons oder sonstige Formkörper gleichzeitig aus einem Schlauchrohling hergestellt werden, indem eine Hohlform mit einer entsprechenden Vielzahl von ballonformenden Kompartimenten verwendet wird, und ein Schlauch-Rohling durch all diese in einer gemeinsamen Flucht hintereinander liegenden, seriell angeordneten Hohlformabschnitte hindurch gespannt und in diese hinein geformt wird. Nach Fertigstellung einer entsprechenden Anzahl seriell augeformter Ballone können dieselben nach dem Entformen voneinander getrennt werden. Die Ausbeute bezogen auf den Materialeinsatz kann so optimiert werden. Eine serielle Anordnung sowie ggf. die sequentielle Ausformung von Ballons aus einem einzigen Rohschlauchelement wird besonders durch einen modularen Aufbau der erfindungsgemäßen Vorrichtung begünstigt.

Parallele Fertigung:

Zur weiteren Steigerung des Auswurfs pro Fertigungszyklus können neben der seriellen Ausformung innerhalb eines Rohschlauchstranges mehrere solche Stränge, parallel zueinander angeordnet und simultane Streckung auf einer gemeinsamen Streckvorrichtung simultan geblasen und temperiert werden (parallele Blasformung bzw. Kombination von serieller und paralleler Blasformung).

Die Erfindung läßt sich dahingehend weiterbilden, dass der Formgebungsvorgang (Einführen eines Rohlings, Vorwärmen, Streckung, Aufblähen, Entspannung, Kühlung und Ausstoß) ausschließlich zeitgesteuert abläuft. Da - im Gegensatz zur konventionellen Technik - die Temperatur der Hohlform bereits nach einem sehr kurzen Zeitraum von einigen Sekunden im thermischen Gleichgewicht mit dem eingefüllten Medium steht und in sämtlichen Segmenten die gewünschte Temperatur angenommen hat. Auf eine Temperaturerfassung an der Hohlform samt übergeordneter Temperaturregelschleife - wie bisher notwendig - kann daher verzichtet werden.

Die masse-technische Ausführung der vorgeschlagenen Hohlform, bzw. das Verhältnis der Wärmekapazitäten zwischen dem Füllvolumen des Mediums und der Hohlform soll dabei so gewählt werden, dass sich Erwärmung und Kühlung auf vorzugsweise jeweils 5 bis 30 Sekunden verkürzen.

Formbau:

Es hat sich als günstig erwiesen, dass die Hohlform aus einem Metall besteht, bspw. aus Aluminium, Kupfer, Eisen, Stahl oder einer Legierung dieser Materialien. Ebenfalls verwendbar sind wärmeleitfähende Kunststoff- oder Karbonverbindungen oder Keramiken. Metall leitet einerseits die Wärme besonders gut und kann daher sehr schnell die Temperatur des Heiz- bzw. Kühlmediums übernehmen; außerdem hat es eine ausreichende mechanische Stabilität, um trotz einer geringen Wandstärke einem ggf. auftretenden Überdruck des in den Hohlraum einschießenden Mediums genauso widerstehen zu können wie thermischen Spannungen aufgrund unterschiedlicher Temperaturen gegenüber der restlichen Berandung des Hohlraums, oder einem inneren Überdruck infolge eines hohen Luftdrucks beim Aufblasen des Ballons. Schließlich sind die meisten Metallsorten auch in elektrisch leitfähige und können daher Wirbelströme führen, so dass zum Beheizen evtl. auch ein elektromagnetisches Wechselfeld verwendet werden kann:

Die Hohlform sollte eine oder mehrere Öffnungen aufweisen, so dass beim Aufblähen des Rohlings Luft, die sich möglicherweise in der Hohlform durch den sich in sie hinein entwickelnden Ballon staut, aus der Hohlform entweichen kann. Natürlich dürfen diese Öffnungen nicht in den hinter der Hohlform liegenden Hohlraum münden, sondern sind von diesem vollständig getrennt, und sind stattdessen mit einer Leitung, bspw. einem Kanal, Schlauch od. dgl., verbunden, der durch den Hohlraum hindurch führt und nach außen mündet.

Es liegt im Rahmen der Erfindung, dass mehrere Öffnungen einer Hohlform an deren Außenseite miteinander kommunizieren, bspw. durch einen Kanal und/oder Schlauch, so dass nur eine einzige Luftauslaßleitung erforderlich ist.

In Weiterbildung dieses Erfindungsgedankens kann vorgesehen sein, dass eine Hohlform durch zwei flächig aneinander liegende Flachkörper gebildet ist, von denen der dem Rohling zugewandte Hohlform-Flachkörper mehrere durchgehende Öffnungen aufweist, der dem Rohling abgewandte Hohlform-Flachkörper dagegen ein System von Sammelkanälen an seiner Innenseite und allenfalls einen oder mehrere Zu- und/oder Ableitungsanschlüsse. Durch Zusammensetzen bzw. -stecken zweier solcher Flachkörper entsteht sodann an der Berührungsfläche ein System von Kanälen, welches die Öffnungen des Innenliegenden Körpers mit einem oder mehreren Zu- und/oder Ableitungsanschlüssen verbindet. Dabei können die Sammelkanäle wahlweise an der Innenseite des äußeren Hohlform-Flachkörpers' oder aber an der Außenseite des inneren Hohlform-Flachkörpers angeordnet sein. Bei dieser Ausführungsform ist wichtig, dass die beiden Flachkörper möglichst großflächig und fest aneinanderliegen, damit der Wärmeaustausch zwischen diesen möglichst nicht behindert wird. Am besten dürfte es daher sein, wenn die Flachkörper nach Art einer Preßpassung zusammenzufügen sind.

Aus fertigungstechnischen Gesichtspunkten kann eine Hohlform unterteilt sein, bspw. in zwei Hälften, die in axialer Richtung des Schlauch-Rohlings auseinander bewegbar sind, ggf. aber auch in einander entgegengesetzten Radialrichtungen. Dadurch ist für den Fall eines Fehlers, bspw. beim Reißen eines Ballons, ein Zugang zum Innenraum der Hohlform gegeben, so dass diese bspw. leicht gereinigt werden kann.

Auch erlaubt dies eine Weiterbildung dahingehend, dass jede solche, in einander entgegengestzten Radialrichtungen auseinanderfahrbare Formhälfte modular aufgebaut ist, mit einem Chassis, woran die eigentliche Formhälfte (im Folgenden als "Inlay" bezeichnet) lösbar festgelegt ist, bspw. angeschraubt. Um ein Austreten des Temperierungsmedium zu vermeiden, kann entlang der Stoßfuge eine Dichtung vorgesehen sein, bspw. ein in die Stoßfuge eingelegter, rundumlaufender Dichtungsring. Wenn - wie die Erfindung weiterhin vorsieht - die eigentliche Formhälfte bzw. das eigentliche Inlay aus Metall besteht, vorzugsweise aus Stahl, insbesondere aus Edelstahl, bspw. mit einer Stärke von 1 bis 3 mm, so ist die Formstabilität selbst bei erhöhtem Fluiddruck ausreichend, um auf diesem Weg Ballons mit einem Durchmesser bis zu etwa 3 cm herzustellen. Will man gleichzeitig an der Außenseite der Form die Temperatur an der Innenseite der Form ablesen, so kann eine mit dem Inlay bspw. identische Form an der Außenseite eines dann rahmenförmigen Chassis angeordnet werden, so dass innen und außen identische Verhältnisse herrschen. Ggf. kann anstelle einer identischen Form an der Außenseite auch eine zu der Innenseite hinsichtlich des Querschnitts übereinstimmende oder dazu spiegelbildliche Form verwendet werden.

Sofern zwei Teile der Hohlform symmetrisch zueinander aufgebaut sind, können sie substantiell dieselbe Geometrie aufweisen, was sich förderlich auf deren Herstellung auswirkt.

Damit die Teile einer Hohlform auch in mit einem Medium befülltem Zustand und damit kurzfristig voneinander getrennt werden können, sind sie an ihrer Außenseite jeweils von einem eigenen, abgeschlossenen Hohlraum umgeben.

Wenn die Stirnseiten des abgeschlossenen Hohlraums eines Hohlformteils druckfest ausgebildet sind, so können sie einfach durch eine axial bzw. parallel zu dem Schlauch-Rohling gerichtete Druckkraft festgelegt und sodann bei Bedarf genauso schnell auch wieder auseinander gefahren werden.

In Weiterverfolgung dieses Erfindungsgedankens sieht die Erfindung vor, dass an der/den oder nahe der Stirnseite(n) eines abgeschlossenen Hohlraums eines Hohlformteils (jeweils) ein oder mehrere Elemente zum Zentrieren und/oder Verriegeln an benachbarten Hohlformteilen vorgesehen sind. Es kann sich hierbei um in axialer Richtung ineinander steckbare Elemente handeln, bspw. mit sich in axialer Richtung konisch bzw. kegel- oder pyramidenförmig verjüngenden Mantelflächen, so dass sie sich beim Zusammenschieben selbsttätig zentrieren.

Ferner sollte ein abgeschlossener Hohlraum zwei Anschlüsse aufweisen zum Ein- und Ausleiten eines Heiz- oder Kühlmediums. Vorzugsweise werden abwechselnd zwei Medien verwendet, die sich auf unterschiedlichen Temperaturniveaus befinden, aber die Strömungsrichtung ist bei beiden Medien dieselbe, so dass ein Anschluß stets als Zulauf und der andere stets als Ablauf dient. Damit wird erreicht, dass ein aus dem Hohlraum hinausgeschobener Teil eines Mediums durch entsprechende Schaltung der Flussregulierenden Ventile wieder dem Reservoir zugeführt werden kann, dem er ursprünglich entnommen wurde, wodurch ungewollte Vermischung in den Reservoirs vermieden werden kann.

Prinzipiell sind drei Füll- bzw. Perfusionszustände der Hohlform denkbar. Vollständige Füllung mit Medium einer Temperatur ohne weitere Perfusion, intermittierende sowie kontinuierliche Perfusion mit einem Medium bestimmter Temperatur. Diese Zustände werden durch geeignete Ventilschaltung im Zufluss zur Hohlform ermöglicht.

Es hat sich bewährt, einem abgeschlossenen Hohlraum eine ringförmige Struktur zu erteilen, so dass sich besonders einfache geometrische und dabei mechanisch stabile Verhältnisse ergeben. Solchenfalls bietet sich eine Weiterbildung dahingehend an, dass ein abgeschlossener Hohlraum an seiner Außenseite durch eine Zylindermantelfläche begrenzt wird, also bspw. entsprechend der Form eines Rohrs.

Formwerkzeug Perfusionsraum:

- ein einziger Hohlraum (bei kompatiblen Medien)
- zwei von einander getrennte Hohlräume (bei inkompatiblen Medien)
- durchgehender Raum (konturähnliche bzw. konturparallele, ineinandergeschachtelte Elemente - Zerspanungstechnik)
- räumlich komplexes in eine einzige Wandungslage integriertes Perfusionsnetz (Laser Cusing)

Formwerkzeug - Perfusionsmodus:

- repetierende Füllung mit intermittierender homogener Durchwärmung im Pausenzustand: bei größerer Formteilmasse
- kontinuierliche Perfusion: bei entsprechend geführten Perfusionskanälen, entweder auf simultan homogene oder zeitlich verschobene segmentorientierte Erwärmung ausgelegt

Ein erfindungsgemäßes Verfahren zur Herstellung von Ballon- oder Hohlkörpern aus einem schlauchförmigen Rohling durch Blasformen mit wenigstens einer temperierbaren Hohlform, innerhalb welcher der Rohling bis zu seiner gewünschten Gestalt aufgebläht wird, zeichnet sich dadurch aus, dass ein flüssiges, heizendes Medium durch ein oder mehrere Hohlräume zirkuliert, der/die sich etwa mittig zwischen diesen Oberflächen des Formwerkzeugs befindet (-en), so dass der in die Werkzeugwandung integrierte medium-führende Innenraum nach außen und Innen von einer Wandung jeweils identischer Stärke abgegrenzt ist, also einen symmetrischen Wandaufbau aufweist, und wobei der strukturelle Aufbau und die Abmessungen der nach innen gerichteten Wandung des Formwerkzeugs zu der Form seiner nach außen gerichteten Wandung so weit wie möglich kongruent oder symmetrisch ist, wobei durch den symmetrischen Wandaufbau des Formwerkzeuges im Verbund mit der Wiedergabe der Forminnenkontur auf der Formaußenwandung die Temperaturentwicklung auf dessen Außenseite der Temperaturentwicklung auf dessen Innenseite nahezu entspricht und so die Ablesung des Temperaturverlaufs, wie er sich an der Forminnenfläche darstellt, auf der Formaußenfläche allseitig möglich macht, wobei an der gegenüberliegenden Fläche der Formaußenwandung die unmittelbare wirksame Formungstemperatur an der Forminnenwandung des Formwerkzeugs, wie sie auf den zu formenden Kunststoff einwirkt, z. B. durch Infrarot-Bildgebung über alle Flächen simultan, in quasi indirekter Weise, dargestellt und im Verlauf beobachtet und erfasst wird, insbesondere in Echtzeit.

Dabei überträgt das eingefüllte Medium einen (kleinen) Teil seiner Wärmemenge auf die Hohlform, es findet innerhalb weniger Sekunden ein Temperaturausgleich statt, Da nur eine vergleichsweise geringe Wärmemenge übertragen wird, um den sehr dünnwandigen Formkörper zu erwärmen bzw. zu kühlen, ist das thermische Gleichgewicht in einem sehr kurzen Zeitraum erreicht, die Zykluszeit, welche maßgeblich durch die Zeitintervalle zum Aufwärmen bzw. vollständigen und gleichförmigen Durchwärmen der Hohlform von der Entnahme-Temperatur auf die Formungs-Temperatur einerseits und zum späteren Abkühlen zurück auf die Entnahme-Temperatur andererseits bestimmt wird, kann daher selbst bei großen Ballonformen (beispielsweise bis zu 10 cm messenden Durchmessern) und aufwendigen Ballongeometrien (beispielsweise Ballonformen mit Segmenten deutlich unterschiedlicher Segmentdurchmesser) auf einen Bruchteil der bisher dafür benötigten Zeit reduziert werden, bspw. auf Zeiten deutlich unterhalb einer Minute, so dass - in Verbindung mit einer Automatisierung der Beschickung und Entformung - eine bedeutend rationellere und damit wirtschaftlichere Herstellung möglich ist als bisher.

Maßgebend hierfür ist u.a., dass die Gesamt-Wärmekapazität der nicht befüllten Hohlform (deutlich) kleiner ist als die Wärmekapazität des den Hohlraum vollständig ausfüllenden Volumens des Temperierungsmediums. Vorzugsweise entspricht die Wärmekapazität der Hohlform nur einem Bruchteil der Wärmekapazität des ausfüllenden Temperierungsmediums, beispielsweise nur der Hälfte oder weniger, vorzugsweise einem Viertel oder darunter, insbesondere einem Zehntel oder einem noch kleineren Bruchteil.

Ferner kann der Hohlraum in der Wandung der Hohlform in zeitlichen Abständen, intermittierend mit demselben Medium gespült werden, um die Temperatur der Hohlform konstant zu halten. Da bei zur Ausformung eventuell erforderlichen längeren Verweilzeit eines Mediums in dem Hohlraum hinter der Hohlform auch Wärmeenergie an die sonstige Berandung des Hohlraums abgegeben wird, kann ein daraus resultierender Wärmeverlust durch ein Ausspülen einer Füllcharge mit einer weiteren Füllung ausgeglichen werden. Eine andere Maßnahme zur Reduzierung eines solchen nach extern gerichteten Temperaturabfalls könnte darin bestehen, nur den die Hohlform bildenden Teil der Berandung des Hohlraums aus einem gut wärmeleitenden Material herzustellen, die übrigen Bereiche der Berandung, nämlich den Außenmantel und die Stirnseiten, dagegen aus einem schlechten Wärmeleiter bspw. einem Kunststoff auszuführen.

Entlüftungskanäle:

- Vakuumunterstützung der Formentlüftung

Schließlich entspricht es der Lehre der Erfindung, dass das Aufblähen des Ballons durch Evakuierung des Hohlraums zwischen dem sich aus dem Rohling entwickelnden Ballon und der Hohlform unterstützt wird. Abhängig von den jeweiligen geometrischen Details des auszuformenden Ballons kann die Ausdehnung des Schlauch-Rohlings durch nicht oder zu langsam abfließendes zu verdrängendes Luftvolumen durch dafür in der Wandung der Hohlform vorgesehene Öffnungen zur Ruptur oder zur fehlerhaften Formung des Ballons führen. Durch eine angeschlossene Vakuumpumpe, welche über die Entlüftungsöffnungen aktiv solches Stauvolumen absaugt, können derartige Effekte vermieden werden. (Bündelung der Entlüftungskanäle, Konnektion mit einem Vakuum erzeugenden Element)

- Detektion des Ballon-Ausbruchs

Bündelung der Entlüftungskanäle, Konnektion mit Drucksensor, beim pop-up des Ballons innerhalb der Form kann ein passagerer Druckanstieg oder momentaner Fluß festgestellt werden.

Es hat sich als günstig erwiesen, dass die Umformung zur Erreichung einer gleichmäßigen radialen Dehnung innerhalb eines begrenzenden Rohres stattfindet.

Ferner hat es sich als günstig erwiesen, dass sich das begrenzende Rohr während oder nach axialer Streckung über den ggf. in Streckung befindlichen Schlauch vorzugsweise halbschalenartig schließt.

Weiterhin hat es sich als günstig erwiesen, dass der Schlauch während der Formgebung bis einschließlich der Entformung nicht oder nur bereichsweise, d.h. teilweise, erhitzt wird, so dass der Schlauch oder zumindest Bereiche desselben kalt verformt wird.

Ferner entspricht es der Lehre der Erfindung, dass Bereiche des Schlauchs kalt verformt werden, andere dagegen heiß.

Die Offenbarung zeichnet sich weiterhin aus durch eine partikel- und keimarme Massenfertigung von Ballonkörpern in einer Reinraum-Produktionsumgebung.

Weitere Vorzüge ergeben sich dadurch, dass die gesamte Temperierung des Formwerkzeugs durch Zuführung flüssiger Temperierungsmedien von einem zentral versorgenden, steuernden und regelnden Modul außerhalb des Reinraum-Fertigungsbereichs erfolgt.

Die jeweils im Werkzeug zu erreichende Temperatur kann durch Fluten von Hohlräumen desselben mit einem Medium definierter Temperatur direkt, präzise, mit nur geringer zeitlicher Verzögerung sowie einem technisch und regeltechnisch insgesamt niedrigen Aufwand, erreicht werden, ggf. unter vollständigem Verzicht auf eine Temperaturfühlung im Werkzeug, wobei die zuvor beschriebene ballistische Temperierung über einen Temperaturgradienten entfällt.

Ferner hat es sich als günstig erwiesen, dass durch die Formteiltechnologie auch die Kombination nicht kompatibler Temperierungsmedien möglich ist.

Anwendung im In-Line Verfahren:

Das beschriebene Prinzip ist sowohl auf das Zwei-Schritt Verfahren (aktive Streckung eines vor-extrudierten Rohlings) als auch für das Ein-Schritt Verfahren geeignet, wobei bei Letzterem eine eigentliche Streckung des frischen Extrudates nicht erfolgt, stattdessen erfolgt die Ausformung aus der Schmelze in einer senkrecht angeordneten Blasform, wobei das Extrudat von oben her fallend sozusagen in die Form hinein abtropft.

Ein offenbartes Verfahren zeichnet sich dadurch aus, dass ein vorextrudierter Rohschlauch in einer einseitig oder beidseitig angeordneten Streckvorrichtung an beiden Schlauchenden fixiert wird; über den axial wirkenden Zugmechanismus wird der Schlauch in Längsrichtung gedehnt; das Schlauchlumen wird über entsprechende Aufblasdorne, über welche der Rohschlauch zugfest geklemmt ist, im Anschluss oder zeitgleich mit der axialen Streckung mit hohem Druck beaufschlagt, so dass sich der Rohschlauch zusätzlich zur axialen Streckung auch radial aufdehnt.

Eine zur Durchführung dieses Verfahrens geeignete Vorrichtung umfaßt eine Klemmvorrichtung zum Festklemmen der Enden des zu verformenden Schlauchs, wenigstens eine Streckvorrichtung, um den Abstand zwischen den Klemmvorrichtungen zu erhöhen, sowie wenigstens einen Aufblasdorn innerhalb eines Schlauchs im Bereich einer Klemmvorrichtung, um den Schlauch mittels Überdruck aufzudehnen.

Zur gleichförmigen radialen Aufdehnung des Schlauchs kann ein begrenzendes Rohr verwendet werden. Ein solches, begrenzendes Rohr braucht nicht oder allenfalls bereichsweise beheizbar sein.

Im Rahmen der Erfindung wird Silikon durch Basismaterialien mit vorzugsweise geringer Volumendehnbarkeit (Compliance) wie z.B. Polyurethan (PUR) oder ein Material mit ähnlichen technischen Elastizitäts-, Formungs- und Festigkeitseigenschaften ersetzt.

Im blow-molding-Verfahren hergestellte Schlauch- und Ballonfolien aus derartigen, wenig volumendehnbaren Materialien, werden im Formungsprozess einer hohen bis maximalen axialen und radialen Dehnung zur Wandstärkereduktion unterzogen. Sie bilden in der Regel sehr glatte Oberflächen von hoher Ebenheit aus und weisen trotz geringer Wandstärken eine ebenfalls hohe Reißfestigkeit auf.

Die erreichte Oberflächengüte erschwert die Kolonisation und erleichtert die mechanische Reinigung der stuhlexponierten Oberflächen.

Vollständig ausgeformte rektale Ankerballonfolien gestatten zudem den Ballon ohne Aufdehnung seiner Hülle und damit einhergehenden nicht gewebeverträglichen Fülldrucken zu entfalten. Typische Ulcera im Rektum können durch einen entsprechend drucklosen Ballonkörper so vermieden werden.

PUR, wie auch LDPE und PVC, erweist sich zudem deutlich besser geruchsdichtend als das bekanntermaßen geruchs- und flüssigkeitspermeable Silikon. ,

PUR gestattet neben der thermischen Verformung eines extrudierten Rohschlauches zu einer dünnwandigen Ballonstruktur im hot-blow-molding Verfahren ebenfalls eine nicht reversible, nicht-thermische Verformung.

Durch axiale Streckung des Schlauchrohlings, Beaufschlagung mit hohem Druck und einer resultierenden maximalen Dehnung des Polymers kann eine inkomplette Retraktion des quasi "überdehnten" Schlauches nach Druckentlastung erreicht werden. Der so dauerhaft verformte Schlauch kann einen Durchmesser und eine Wandstärke erreichen, die beispielsweise seine Verwendung als drainierender Zwischenschlauch in Stuhldrainagesystemen typischen Aufbaues gestattet.

Durch die maximale Dehnung des zu verformenden Rohschlauches in radialer und axialer Richtung wird bei der kalten Verformung ein polymerer Orientierungsgrad erreicht, der das verformte aufgedehnte Schlauchelement mit hoher Zug- und Reißfestigkeit der Schlauchfolie ausstattet.

Die durch das vorgestellte Verfahren beschriebene Kombination von hohem Schlauchdurchmesser, geringer Folienschlauchwandstärke, Weichfoliencharakteristika, Oberflächengüte und gleichzeitig hoher mechanischer Stabilität sind durch primäre Extrusion oder andere Herstellungsverfahren in der Regel nicht erreichbar. Die Erfindung bietet sich daher generell zur Herstellung von Schlauchfolien mit entsprechenden Charakteristika auch in anderen medizinischen oder auch sonstigen technischen Anwendungsbereichen an.

Schließlich beschreibt die Offenbarung den kalten Überdehnungsprozess zudem in kombinierter Anwendung mit dem hot-blow-molding-Verfahren. Eine derartige Kombination gestattet zum Beispiel bei der Herstellung von Stuhldrainagesystemen die kombinierte simultane Fertigung des rektalen Ankerballons sowie der sich nach proximal zum Beutel hin anschließenden drainieren Segmente des Produktes aus einem einzigen Schlauchrohling.

Weitere Merkmale, Einzelheiten, Vorteile und Wirkungen auf der Basis der Erfindung ergeben sich aus der folgenden Beschreibung bevorzugter Ausführungsformen der Erfindung sowie anhand der Zeichnung. Hierbei zeigt:

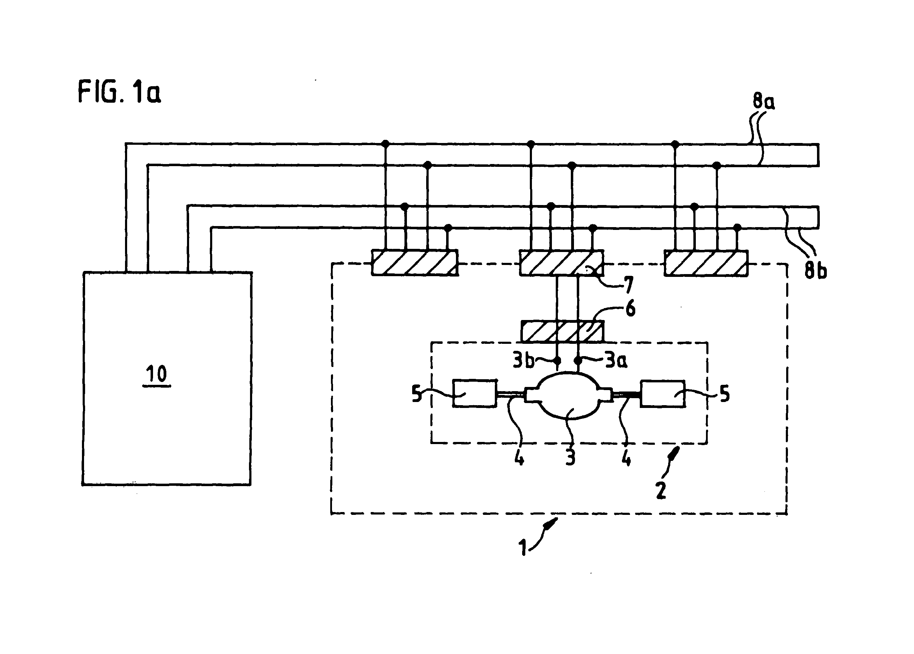

Fig. 1a     eine schematische Darstellung der Einbindung der Form in eine Gesamtfertigungsanlage;

Fig. 1     b eine Ausführungsform der Gesamtanordnung, bei der die von extern in den Reinraum geführten Versorgungsstränge in einer zentral angeordneten Versorgungssäule münden (seitliche Ansicht);

Fig. 1c     die Anordnung nach Fig. 1b in einer Ansicht von oben;

Fig. 2a,2b     eine schematische Darstellung der Blasformeinheit;

Fig. 3a-3c     eine schematisierte Darstellung einer Blasform, sowie mehrere beispielhafte Ausführungsformen von medium-führenden Kanalformationen innerhalb der Formwandung;

Fig. 4a,4b     ein Formwerkzeug mit fensterartigen Öffnungen zur infrarotoptischen bzw. berührungsfreien, großflächigen Erfassung des Temperaturverlaufes, wobei Fig. 4a einen Längsschnitt und Fig. 4b eine Draufsicht darstellen;

Fig. 5     eine typische Ausführungsform einer Anordnung zur Stuhldrainage;

Fig. 6     den schematischen Aufbau einer Verformungsvorrichtung zur Herstellung einer Anordnung aus Fig. 5;

Fig. 7     eine andere Ausführungsform der Erfindung zur Herstellung einer Anordnung aus Fig. 5;

Fig. 8     einen Längsschnitt durch eine wiederum abgewandelte Ausführungsform der Erfindung;

Fig. 9     den zeitlichen Verlauf der Temperatur während des Blasformens, eines Ballon- oder Hohlkörpers mittels der Vorrichtung aus Fig. 8;

Fig. 10     einen Schnitt quer durch zwei Hälften eines entlang seiner Längsachse geteilten Formwerkzeugs; sowie

Fig. 11     eine der Fig. 10 entsprechende Darstellung einer wiederum abgewandelten Ausführungsform der Erfindung.

Die Anlage 1 aus Fig. 1a dient zum Herstellen von Ballons oder Hohlkörpern für die Medizintechnik, insbesondere für Ballonkatheter od. dgl., wobei schlauchförmige Rohlinge, vorzugsweise aus Polyurethan, in die gewünschte Ballonform aufgeblasen werden.

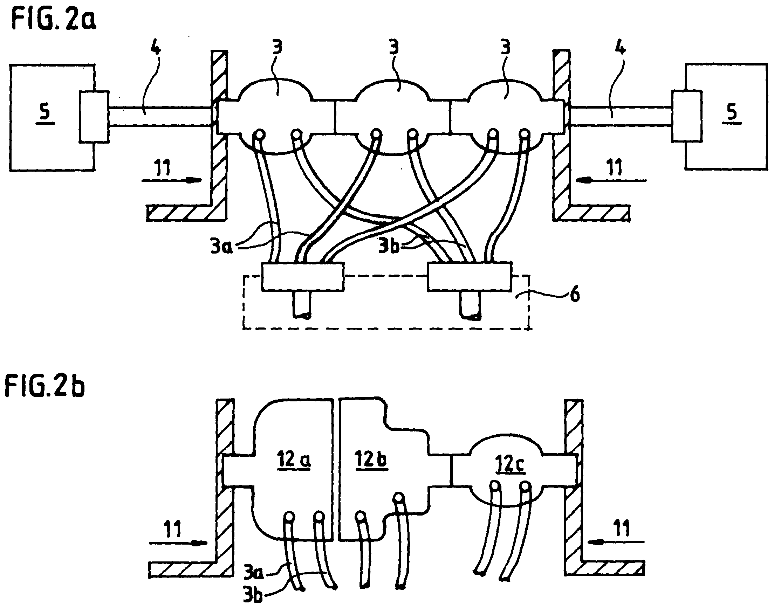

Den Kernpunkt der Anlage 1 bildet die eigentliche Blasformeinheit 2, die schematisch auch in Fig. 2 wiedergegeben ist.

Das Blasformwerkzeug 3 nimmt den vorextrudierten Rohschlauch 4 auf. Dieser wird durch eine Streckvorrichtung 5 axial gestreckt und anschließend mit einem Blasdruck beaufschlagt, der den Schlauch 4 innerhalb der Form 3 ballonartig austreibt und, in der Regel unter erheblicher Spannung, an die innere Formwandung des Werkzeuges 3 schmiegt. Durch Erwärmung und anschließende Kühlung wird der aus dem Schlauch 4 ausgeformte Körper in seiner Form fixiert.

Die Blasformeinheit 2 verfügt im idealen Fall nur über die für die Ausformung essentiellen Komponenten, wie eine Streckvorrichtung 5 und ein Verteilerelement 6, an welchem der Vorlauf 3a und der Rücklauf 3b der jeweiligen Formwerkzeuge 3 angeschlossen werden. Dem Verteilerelement 6 vorgeschaltet ist ein Ventilblock 7, der den Zufluss aus den verschiedenen angeschlossenen Versorgungskreisläufen 8a und 8b kontrolliert.

Zur Gewährleistung bestmöglicher hygienischer Bedingungen im Fertigungsbereich 9 werden sämtliche versorgenden Zuleitungen 8a, 8b vorzugsweise außerhalb des Bereiches 9 geführt. Die Verbindung zu einer oder mehreren, modular anschließbaren Fertigungseinheiten 2 erfolgt über entsprechend ausgelegte Docking-Stationen, die jeweils im Wesentlichen dem Ventilblock 7 entsprechen, aber auch elektrische Anschlüsse und Druckluftverbindungen enthalten können.

Der eigentliche Fertigungsbereich 9 ist als Reinraum ausgeführt, um Partikel und sonstige Verunreinigungen in der Luft von der/den Blasformeinheit(en) möglichst fernzuhalten und auch aus hygienischen Gründen. Von dem Reinraum 9 räumlich getrennt, d.h. außerhalb desselben, werden die rein versorgenden Funktionsteile, wie Spannungsversorgung, Pneumatik-Versorgung, Temperierung der einzelnen Versorgungskreisläufe und Prozess-Steuerung bzw. -statistische Dokumentation in einer zentralen Einheit 10 zusammengefasst.

In der einfachsten Ausführung verfügt das versorgende Kreissystem nur über ein einziges Temperierungsniveau, welches exakt auf die eigentliche Formtemperatur eingestellt ist. Die Kühlung kann durch ein lokal zugeführtes oder erzeugtes, und am Ort der Verwendung verworfenes Medium, wie z.B. Leitungswasser oder Pressluft erfolgen.

Vorteilhaft wird die Kühlung jedoch in Form eines flüssigen Mediums im Kreissystem bereitgestellt, idealerweise als geschlossenes System, welches nach extern über einen Wärmetauscher kommuniziert. Beispielsweise können in beiden Kreisläufen identische Thermo-Öle oder jeweils Wasser zirkulieren.

Beim Blasformen spezifischer Materialien können allerdings mehrere Hochtemperatur-Niveaus erforderlich sein. Beispielsweise erfordert die Blasformung von Polyurethan (PUR) zwei Temperaturniveaus, wobei die obere Temperatur der zur Ausformung und völligen Entspannung erforderlichen Temperatur entspricht, in der Regel bei Polyurethan etwa 160 bis 170 Grad Celsius, die untere Temperatur hingegen sowohl für die Entnahme des Ballons aus der Form geeignet ist als auch für die Vortemperierung des Schlauchrohlings verwendet werden kann. Diese Temperatur liegt vorzugsweise bei 50 bis 60 Grad.

Sind darüber hinaus weitere Hochtemperatur-Niveaus erforderlich, können diese dem Prozess über entsprechende Zuleitungen von der zentralen Einheit her zugeführt werden.

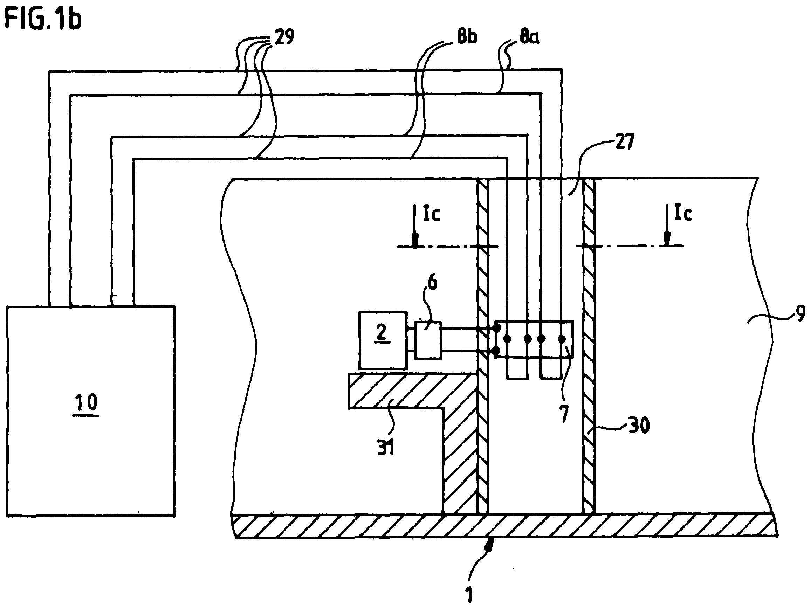

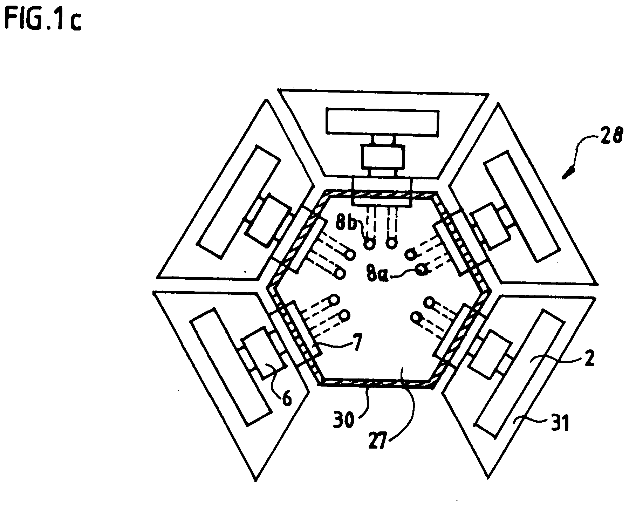

Fig. 1b und 1c zeigen, als Alternative zu einer ringförmig um den Reinraum herum angelegten Führung der Versorgungsleitungen, eine inselartige Anordnung mehrerer Formungseinheiten 2 um eine zentrale Versorgungssäule 27. Formungseinheiten und Versorgungssäule bilden sozusagen eine Fertigungsinsel 28. Die Versorgungssäule 27 ist dabei vorzugsweise derart konstruiert, dass über ihre Außenflächen 30 die variable Ankopplung von individuellen Fertigungsmodulen 2 möglich ist, so dass an einer einzigen Fertigungsinsel 28 ggf. mittels unterschiedlicher Fertigungsmodule 2 unterschiedliche Ballons angefertigt werden können. Die Fertigungsmodule 2 können dabei auf je einem Tisch 31 aufgesetzt werden, der sich von der betreffenden Seite 30 der Säule 27 etwa radial nach außen erstreckt.

Die Zuführung der Versorgungsleitungen von einer externen Zentraleinheit kann wahlweise über eine am und/oder im Bereich der Raumdecke und/oder des Bodens geführte Trasse 29 erfolgen. Die versorgende Säuleneinheit 27 kann derart gestaltet sein, dass sie beispielsweise über sechs Kanten verfügt, wobei ggf. fünf der durch die Kanten gebildeten Flächen eine Andockung jeweils einer Formungseinheit an die betreffende Säulenfläche ermöglichen, wodurch sich insgesamt eine rosettenartige Verteilung der Fertigungsmodule 2 ergibt. Die sechste Fläche 30 kann frei bleiben und gestattet den Zugang zu den Installationen im Säuleninneren. Die Versorgungswege zu den Fertigungseinheiten können bei der vorgeschlagenen rosettenartigen Anordnung optimal verkürzt werden, bei gleichzeitiger Maximierung der Anzahl der möglichen anschließbaren Fertigungsmodule 2. Eine derartige rosettenartige Anordnung um eine versorgende Zentralsäule bietet den weiteren Vorteil, dass eine Bedienperson bequem von einer Formungseinheit 2 zur nächsten wechseln kann.

Die Säule 27 kann bevorzugt auch den flußregulierenden Ventilblock 7 der jeweiligen Formungseinheit fest integriert (verrohrt) in sich aufnehmen, so dass zum Anschluss an eine variable Formungseinheit 2 neben elektrischen Verbindungen und Druckluftverbindungen nur lediglich zwei Schlauchverbindungen, insbesondere Vor- und Rücklauf, erforderlich sind.

Fig. 2 beschreibt die Blasformeinheit 2 im Detail. In Fig. 2a ist die Möglichkeit dargestellt, dass mehrere Blasformwerkzeuge 3 in serieller Anordnung betrieben werden können. Mehrere Formwerkzeuge 3 - im dargestellten Fall drei - sind hierzu sequentiell, d.h. in Längsrichtung der Streckvorrichtung 5 hintereinander, angeordnet und werden durch eine axial wirkende Kraft - verursacht durch eine äußere Spannvorrichtung 11 - aneinander gepresst.

Alternativ können mehrere Werkzeuge 3 auch parallel zueinander angeordnet werden, wobei bei paralleler Anordnung pro Arbeitsstrang jeweils eine separate Fassung des jeweiligen Schlauches an der gemeinschaftlichen Streckvorrichtung 5 vorgesehen ist. Seriell angeordnete Werkzeuge 3 können auch in mehreren parallelen Arbeitssträngen kombiniert werden.

Entsprechend Fig. 2b können bei komplexen, insbesondere mehrfach gekammerten Formen einzelne Formsegmente, wie beispielweise die. Segmente 12a, 12b und 12c hergestellt werden, die sequentiell gefügt und ebenfalls über die zusammenpressende Kraft einer axial wirkenden Spannvorrichtung 11 miteinander verbunden werden.

Das einzelne Werkzeug 3 ist bevorzugt für die Perfusion mit einem einheitlichen Medium konzipiert, welches dem Prozess auf verschiedenen Temperaturniveaus zugeführt wird. Die Medien sind daher vermischbar und erfordern nur einen einzigen Perfusionsraum.

Bei nicht kompatiblen Medien, wie beispielsweise Öl und Wasser, kann ein ZweiKammer-System in die Wandung der Form 3 integriert werden, worin die Medien weitgehend parallel geführt, jedoch räumlich voneinander getrennt sind.

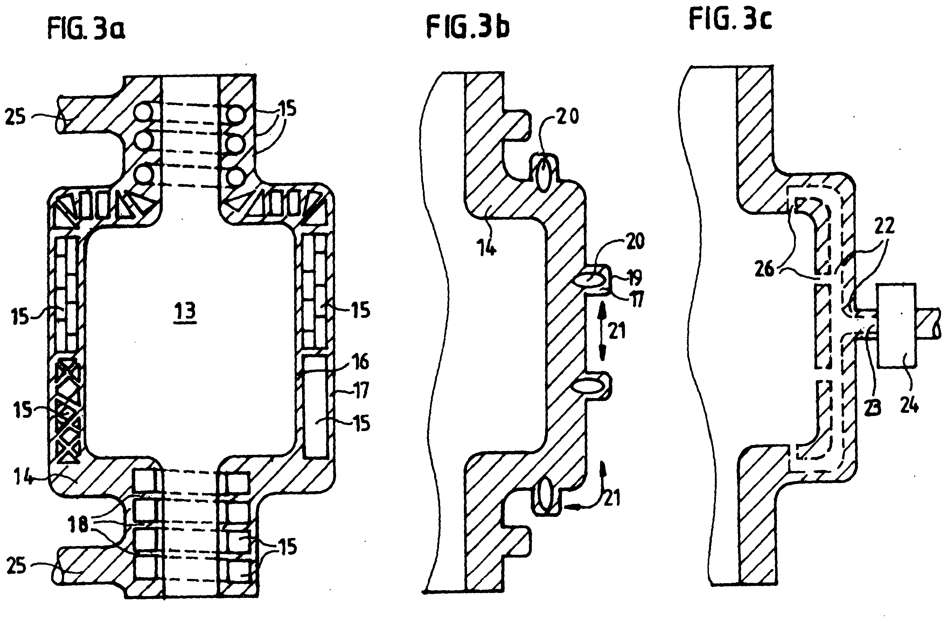

In den Fig. 3a - 3c wird der Aufbau einer Blasform 3 an Hand verschiedener Ausführungsbeispiele erläutert.

Die Form 3 weist einen möglichst symmetrischen Aufbau der den Formungsraum 13 umschließenden Wandung 14 auf. Innerhalb der Wandung 14 ist ein Perfusionsraum 15 ausgeformt bzw. integriert.

Eine besondere Stabilität der Formwandung 14 kann erreicht werden, indem die nach außen und innen weisenden Grenzflächen 16, 17 des Werkzeuges 3 - also dessen Innenseite 16 und Außenseite 17 - zwar dünnwandig ausgeführt sind, jedoch über ein System von zwischen den Grenzflächen 16, 17 liegenden Verstrebungen 18, welche Trabekeln oder Stegen ähneln können, entweder direkt miteinander verbunden sind, oder über parallel zur Wandung 14 im Perfusionsraum 15 verlaufende Zwischenebenen, die beispielsweise wiederum siebartig durchbrochen sind, in Verbindung stehen. Durch eine entsprechend dicht zueinander angeordnete Verstrebung 18 kann die Problematik großflächig wirkender Kraft und die daraus resultierende Erfordernis dickwandiger Grenzflächen auf interessante Weise weitgehend umgangen werden.

Neben aufwendig trabekulär verstärkten, waben- oder schwammartigen Perfusionsräumen, können auch einfache zirkuläre oder beispielsweise rechteckige Temperierungskanäle in Art einer Wendel durch die Formwandung 14 geführt werden. Durch Variation der Kanaldurchmesser kann die lokale Perfusion bzw. der wirksame Fluss und damit Wärmetransport, gezielt beeinflusst werden. Eine Variation der lokalen Wärmeentwicklung kann auch durch entsprechende Variation der Kanalabstände zueinander erreicht werden.

Wie Fig. 3b zeigt, kann bei erforderlicher, lokal besonders intensiver Temperierung über entsprechende Ausformungen 19 der äußeren Wandung 17, in denen entsprechend großlumige zuleitende Kanäle 20 eingearbeitet sind, eine große Menge Temperierungsmedium zugeführt werden.

Die Ausformungen 19 können auch zur weiteren Stabilisierung der Gesamtform 3 bei besonders großen Formen mit entsprechend großen kraftwirksamen Oberflächen auf der Außenwandung 17 dienen und beispielsweise als netzartige Verstrebung über die Formoberfläche angelegt werden, wobei die Ausformungen 19 so zueinander angeordnet und beanstandet sind, dass im Zwischenraum der verstärkenden Streben 19 noch ausreichend große Abschnitte 21 erhalten bleiben, welche die erfindungsgemäße Symmetrie des Wandungsaufbaus aufweisen, und so eine indirekte Beobachtung der Form-Innentemperatur ermöglichen.

Gemäß Fig. 3c kann neben den medienführenden Kanälen 15, 20 auch ein System von Kanälen 22 eingearbeitet sein, welches beim Formungsprozess verdrängte Luft aus der Formhöhle aufnimmt und zur Umgebung hin abführt. Es liegt im Rahmen der Erfindung, dass mehrere Öffnungen 26 zur Formhöhle durch die innere Wandung 16 angelegt sind, zur Außenseite 17 hin jedoch derart miteinander verbunden werden, dass nur ein einziger Luftauslass 23 erforderlich ist. Hier kann zur zeitlichen Erfassung der Expansion des Ballons aus dem Rohschlauch ein Drucksensor 24 angeschlossen sein, der einen korrespondierenden kurzfristigen Druckanstieg im ableitenden System wahrnimmt.

Möglich sind ebenfalls schichtartig, zueinander versetzt angeordnete, besonders dünnwandig ausgeführte Lagen von wabenähnlichen Geflechten, die in ihrer Anordnung zueinander versetzt, einen aufwendig verstrebten Perfusionsraum ermöglichen, bei gleichzeitig geringem Materialeinsatz.

Derartig komplexe verstrebende Strukturen oder auch komplex geführte Perfusionskanäle sind mit herkömmlichen Methoden des Formenbaus nur schwer, sehr kostenintensiv, oder gar nicht herstellbar. Die Methode des Laser-Sinterns (Laser-Cusing) bietet jedoch eine interessante Fertigungsoption. Durch schichtweisen Aufbau von laser-verschweisstem Metallstaub, lassen sich auch aufwendige dreidimensionale Strukturen auf kleinstem Raum herstellen. Komplex kommunizierende, beispielsweise schwammartige Hohlräume sind auf diesem Weg; komfortabel und preisgünstig fertigbar.

Das Verfahren lässt einen extremen Leichtbau zu, der die Gesamtmasse eines Werkzeuges 3 soweit reduziert, dass es möglich ist, sämtliche Oberflächen 16, 17 des Werkzeuges 3 adäquat zu temperieren, ohne dass ein den Wärmefluss antreibender Temperaturgradient erforderlich ist bzw. diesen, bei größeren Formmassen, auf ein geringes, den Ballon nicht durch Übertemperierung gefährdendes Maß zu reduzieren.

Angestrebt werden Materialwandstärken der Wandungen 16 und 17 bei entsprechender gegenseitiger Verstrebung und Verwendung von nichtkorrodierenden Stahl-Typen von ca. 1 bis 2,5 mm. Bei entsprechender Verwendung von Aluminium, Kupfer oder Messing sind Wandungsstärken zwischen 2 und 3,5 mm vorgesehen. Die Höhe des mediumführenden Raumes 15 zwischen den beiden Wandungen 16 und 17 sollte im Bereich von 1 bis 5 mm liegen.

Wird auf Verstrebungen zwischen den Wandungen 16 und 17 verzichtet, sind bei im Raum 15 wirksamen Druckwerten von bis zu 25 bar bei Verwendung von Stahl Wandstärken von ca. 3 bis 6 mm erforderlich, jeweils abhängig von der Größe der verbauten kraftaufnehmenden Flächen im Werkzeug.

Der durchströmte Hohlraum 15 sollte mindestens zwei Anschlüsse 25 zum Ein- und Ausleiten eines Heiz- oder Kühlmediums aufweisen. Vorzugsweise werden abwechselnd zwei Medien verwendet, die sich auf unterschiedlichen Temperaturniveaus befinden, aber die Strömungsrichtung ist bei beiden Medien dieselbe, so dass ein Anschluss 25 stets als Zulauf und der andere stets als Ablauf dient. Damit wird erreicht, dass ein aus dem Hohlraum 15 hinausgeschobener Teil eines Mediums durch entsprechende temperatur- oder zeitgesteuerte Schaltung der flussregulierenden Ventile im Ventilblock 6 wieder dem Kreissystem zugeführt werden kann, dem er ursprünglich entnommen wurde, wodurch ungewollte Vermischung der Medien in den Kreisen vermieden werden kann.

Es hat sich als günstig erwiesen, dass die Hohlform 3 aus Metall besteht, bspw. aus Aluminium, Kupfer, Eisen, Stahl oder einer Legierung dieser Materialien. Ebenfalls verwendbar sind wärmeleitfähige Kunststoff- oder Karbonverbindungen oder Keramiken. Metall leitet einerseits die Wärme besonders gut und kann daher sehr schnell die Temperatur des Heiz- bzw. Kühlmediums übernehmen; außerdem hat es eine ausreichende mechanische Stabilität, um trotz einer geringen Wandstärke einem ggf. auftretenden Überdruck des in den Hohlraum einschießenden Mediums genauso widerstehen zu können, oder einem inneren Überdruck infolge eines hohen Luftdrucks beim Aufblasen des Ballons. Schließlich sind die meisten Metallsorten auch im Laser-Sinterverfahren verarbeitbar.

Das beschriebene Werkzeug 3 ist sowohl auf das Zwei-Schritt-Verfahren (aktive Streckung eines vor-extrudierten Rohlings) als auch für das Ein-Schritt-Verfahren geeignet, wobei bei letzterem eine eigentliche Streckung des frischen Extrudates nicht erfolgt; statt dessen erfolgt die Ausformung aus der Schmelze in einer senkrecht angeordneten Blasform, wobei das Extrudat von oben her fallend sozusagen in die Form hinein abtropft.

Die Vorteile der Zykluszeit-Optimierung als auch die Option der allseitig auf der Formaußenwandung ablesbaren Innen-Formtemperatur sind beim Ein-Schritt Verfahren ebenfalls vorteilhaft umsetzbar.

Entsprechend der Hohlräume 15 des Kanalsystems läßt sich die gesamte Fläche Fg eines Längsschnittes durch die Wandung der Form 3 unterteilen in den eigentlichen Schnittflächenanteil $f_F$ der Form 3 selbst (die metallenen Schnittflächen) und in den auf die Hohlräume 15 des Kanalsystems entfallenden Schnittflächenanteil $f_H$:

$$F_g = f_F + f_H.$$

Damit läßt sich ein Sollwert für den Außenradius R bestimmen zu:

$$f_H \geq f_F * k * (c_1 * ?_1)/ (c_2 * ?_2),$$

wobei:

$c_1$ = spezifische Wärmekapazität der Hohlform 3
$?_1$ = spezifische Dichte der Hohlform 3
$d$ = Dicke der Hohlform 3
$c_2$ = spezifische Wärmekapazität des Mediums in dem Hohlraum 15
$?_2$ = spezifische Dichte des Mediums in dem Hohlraum 15

Der Faktor k sollte gewählt werden zu $k \geq 0,5$, vorzugsweise zu $k \geq 1,0$, insbesondere zu $k \geq 2,0$, damit sich die Temperatur des Mediums in dem Hohlraum 15 beim Aufheizen/Abkühlen der Hohlform 3 nicht wesentlich verändert, denn es gilt näherungsweise für den thermischen Gleichgewichtszustand:

$$T_x = T_1 + (T_2 - T_1) * k/(1 + k)$$

wobei

$T_1$ = Temperatur der Hohlform 3 vor Einleitung eines Mediums in den Hohlraum 15
$T_2$ = Temperatur des Mediums vor Einleitung in den Hohlraum 15
$T_x$ = Mischtemperatur von Hohlform 3 und Medium in dem Hohlraum 15 nach vollständigem Temperaturausgleich.

Bei $k = 1$ liegt $T_x$ mittig zwischen $T_1$ und $T_2$; je größer k gewählt wird, um so näher liegt die Mischtemperatur $T_x$ bei der ursprünglichen Temperatur $T_2$ des in den Hohlraum 15 eingefüllten Mediums.

Dabei trifft diese Abschätzung um so besser zu, je geringer der Wärmeaustausch mit dem in den Hohlraum 15 eingefüllten Medium mit den übrigen Begrenzungsflächen 16, 17 dieses Hohlraums 15 ist. Deshalb ist es sinnvoll, die radial außen liegende Begrenzungsfläche 16 des Hohlraums 15 aus einem thermisch isolierenden Werkstoff anzufertigen oder zumindest an ihrer Innenseite mit einem solchen Material auszukleiden, bspw. mit Kunststoff, so dass an dieser Stelle ein möglichst geringer Wärmeaustausch stattfindet.

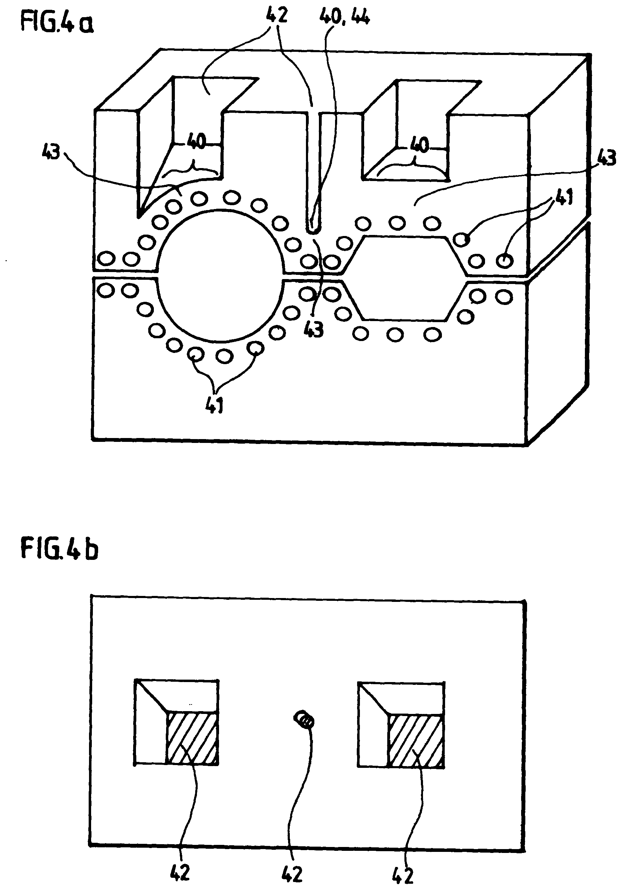

Fig. 4a und 4b zeigen ein Formwerkzeug konventioneller Bauart, welches fensterartige Öffnungen zur infrarot-optischen bzw. berührungsfreien, großflächigen Erfassung des Temperaturverlaufes integriert, wobei Fig. 4a einen Längsschnitt und Fig. 4b eine Draufsicht darstellen.

Konventionelle Formungswerkzeuge für beispielsweise Spritzguss oder Blasformung zeigen in der Regel einen kompakten, massereichen Aufbau, vobei das Werkzeug als Block aus mehreren verstifteten bzw. Ineinandergreifenden Elementen besteht. Die Gesamtanordnung der Formelemente erreicht in der Regel die Geometrie eines einfachen Quaders, Kubus oder Zylinders.

Zwar erfolgt in der Regel die Temperierung über ein in die jeweiligen Elemente integriertes mediumführendes Kanalsystem 41, welches der Formungsinnenwand angenähert ist, eine Symmetrie der formenden mediumtemperierten Wandung, wie erfindungsgemäß bespielsweise in den Figuren 3a bis 3c dargestellt, ist nicht bekannt.

Das Prinzip der Darstellung der Formungstemperatur auf der Formwandaußenseite kann jedoch auch in herkömmliche massive Werkzeugtechnik integriert werden. Beispielsweise kann bestimmten formungskritischen oder temperierungs-repräsentativen Segmenten 40 des Werkzeuges eine erfindungsgemäße Fensterung 42 bzw. Schaffung eines symmetrischen Wandaufbaus 43 hergestellt werden, währen die weitere Außenkontur von der üblichen Massivbauweise nicht abweicht.

Fig. 4b zeigt derartige Mess-Fenster. Die so freigelegten Oberflächen wandsymmetrischer Areale gestatten eine indirekte, für Fertigungszwecke in der Regel ausreichend präzise, großflächige oder auch punktuelle 44 fortlaufende Ablesung der Temperaturentwicklung an der Formungsfläche.

Die Erfassung kann beispielsweise flächig durch Infrarot-Bildgebung oder punktuell durch entsprechend angebrachte Infrarot-Meßfühler erfolgen, wobei Fühler und Messeinheit nicht zwingend am Werkzeug selbst angebracht sein müssen, sondern eine beabstandete, berührungsfreie Messung, quasi in das Werkzeug hinein, erlauben.

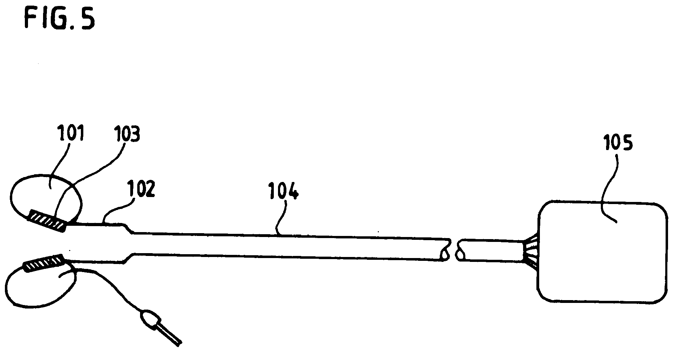

In den Abbildungen 5 bis 7 werden die typische Ausführung einer Stuhldrainagevorrichtung und deren Komponenten vorgestellt sowie erfindungsgemäße Vorrichtungen zum dauerhaften kalten Verformen eines Rohlschlauches zu derartigen, schlauchartigen Segmenten großen Durchmessers und geringer Wandstärke beschrieben.

Wie aus Fig. 5 zu entnehmen ist, werden intra-rektaler Ankerballon 101 und trans-anales Zwischensegment 102 in der Regel aus separaten Komponenten gefertigt. Der intra-rektale Anteil 101 verfügt dabei über ein trichterartiges Mundstück 103, welches die Drainage permanent offen hält und an dem sich der trans-anale Teil 102 direkt anschließt. Der trans-anale Teil 102 geht in einen ableitenden Schlauch 104 über, der in einem beutel-artigen Sammelgefäß 105 endet.

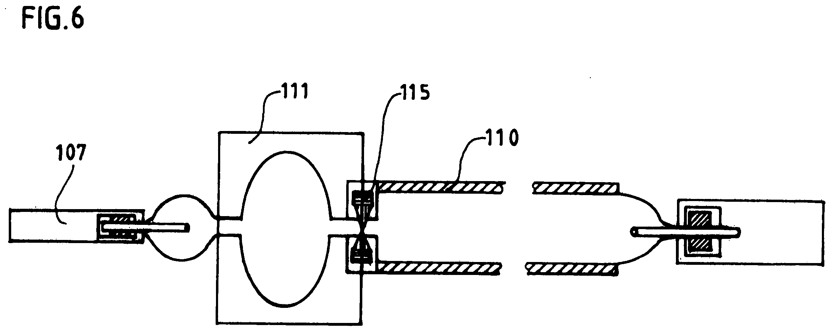

Wie in Fig. 6 zu erkennen, wird ein vor-extrudierter Rohschlauch 106 aus beispielsweise PUR der Sorte Pellethane 2363 70A(E) bis 90A(E) bzw. 60D(E) von Dow Chemical Corp. in einer einseitig oder beidseitig angeordneten Streckvorrichtung 107 an beiden Schlauchenden 108 fixiert. Über den axial wirkenden Zugmechanismus 107 wird der Schlauch 106 in Längsrichtung gedehnt. Der Zugmechanismus kann zum Beispiel durch einen pneumatisch oder elektrisch angetriebenen, axial wirkenden Verfahrschlitten realisiert werden. Das Schlauchlumen wird über entsprechende Aufblasdorne 109, über welche der Rohschlauch zugfest geklemmt wird, im Anschluss oder zeitgleich mit der axialen Streckung mit hohem Druck beaufschlagt. Der Rohschlauch dehnt sich zusätzlich zur axialen Streckung auch radial auf. Zur Erreichung einer gleichmäßigen radialen Dehnung kann die Umformung innerhalb eines begrenzenden Rohres 110 erfolgen, welches sich nach axialer Streckung, durch einen entsprechenden Mechanismus betätigt, beispielsweise halbschalen-artig über den in Streckung befindlichen Schlauch 106 schließt.

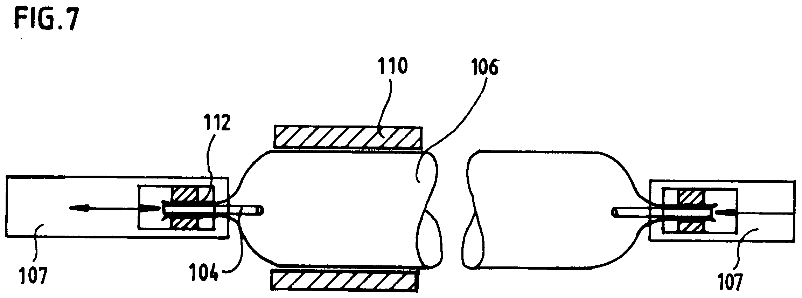

Das Werkzeug 111 aus Fig. 7 kann für die Ausformung des Ankerballons 101 sowie optional zur Ausformung des trans-analen Segmentes 102 bzw. beider Anteile gleichzeitig verwendet werden. Es wird über dem entsprechenden Rohschlauchsegment, neben den nötigen Elementen zur kalten Verformung, in Position gebracht.

Eine kombinierte zeitgleiche kalte und heiße Verformung gestattet die weitestgehend mögliche Reduktion von Einzelkomponenten im Produkt sowie eine bestmögliche Ausbeute des eingesetzten Materials. Im Idealfall können, beispielsweise bei der Fertigung einer Stuhldrainage, mit Ausnahme des Trichterelementes 103, alle Komponenten bis zum Beutel 105 hin in einem Arbeitsgang gefertigt werden.

Die Kombination der kalten und heißen Prozesse gestattet zudem die Ausformung zirkulärer, detailliert gestalteter Wulststrukturen im Bereich der intra-rektalen Kopfeinheit, insbesondere zur Verankerung derselben. Solche Strukturen können auch die Montage und dauerhafte Verbindung zusätzlicher Funktionselemente erleichtern. So kann beispielsweise durch Einsetzen des Trichterelementes 103 in die distale Öffnung des kombiniert geformten Werkstückes eine funktionell günstige und montage-technisch einfache Verbindung der thermisch verschieden geformten Komponenten hergestellt werden.

Um während des Verformungsprozesses eine separate Führung des Druckes im heiß verformten als auch im kalt verformten Abschnitt zu erreichen, kann durch eine Klemmvorrichtung 112 im Übergangsbereich der Verformungsbereiche eine räumliche Trennung hergestellt werden. Die Kompartimente können dann jeweils über einen Aufblasdorn 109 von beiden Seiten der Klemmung der Steckvorrichtung separat befüllt werden.

Neben PUR der zuvor beschriebenen Sorten bzw. Sorten mit vergleichbaren Verformungseigenschaften sind mit dieser Technik auch entsprechend verformte Schlauchfolien aus PVC und LDPE herstellbar. Bei diesen beiden Materialien ist eine kalte Verformung über sämtliche Segmente der Drainage in einem einzigen Formwerkzeug bzw. Arbeitsgang denkbar, was beispielsweise eine kalte Ausformung der intra-rektalen Ballonkomponente einer Stuhldrainage ermöglicht.

Die Wandstärke der intra-rektalen Ballonhülle sollte bei Verwendung aller genannten Materialien im Bereich von 10 bis 40 Mikrometern liegen. Der maximale transversale Durchmesser des intra-rektalen Ballons, bei freier druckloser Entfaltung auf das präformierte Maß, sollte bei ca. 6-9 cm liegen. Die axiale Länge des Ballonkörpers liegt vorzugsweise bei 4-6 cm. Die Verformung dieser Komponente erfordert bei Verwendung von PUR das hotblow-molding Verfahren. Der Rohschlauch wird hierbei durch heiße thermische Behandlung auf große Durchmesser bzw. komplexe Formen hin verformt und anschließend durch Kühlung in der Konfiguration des Formwerkzeuges fixiert.

Die dimensionale Retraktion der geformten Komponente nach der Entnahme aus der Form beträgt in der Regel etwa 10 bis 12 %

Das trans-anale Segment 102 einer Stuhldrainage kann, abhängig von der angestrebten Dimensionierung und der zu realisierenden Strukturdetails sowohl thermisch oder auch nicht-thermisch verformt werden.

Der Drainageschlauch 105 sollte im Sinne einer möglichst patientenfreundlichen Ausführung aus einer dünnwandigen, reißfesten und vorzugsweise nicht bzw. nur wenig dehnbaren Schlauch-Hülle bestehen. Er sollte im ausgeformten Zustand einen Durchmesser von 2,5 bis 4,5 cm aufweisen und im nicht befüllten Zustand spontan zu einem flachen Band kollabieren bzw. mit moderater Kraft zu einem derartigen Band verformbar sein und als solcher sein Inhalt leicht manuell zum Beutel hin ausstreichbar sein. Eine Wandungsstärke von ca. 0,1 bis 0,2 mm ist hierzu bei Verwendung der genannten PUR Sorten besonders geeignet.

Bei Verwendung von PUR der Familie Pellethane 2363 sowie der Härten 70A(E) bis 90A(E) bzw. 60A(E) und einem daraus gefertigten Rohschlauch mit einem Innen-Durchmesser von z.B. 14 bis 16 mm und einer Wandungsstärke von 0.3 bis 0,5 mm, muss beispielsweise wie folgt verfahren werden: Der Rohling wird vorzugsweise um circa 50 bis 150 % seiner Ausgangslänge gestreckt und anschließend mit einem Druck von bis zu 2.5 bar bis zur maximalen radialen Dehnungskapazität, von ca. 6 bis 9 cm aufgedehnt. Ohne Einwirkung von Wärmeenergie kann nach Ablassen des Blasdruckes eine dauerhafte Weitung des Rohlings auf den Zieldurchmesser von ca. 2,5 bis 4,5 cm erreicht werden.

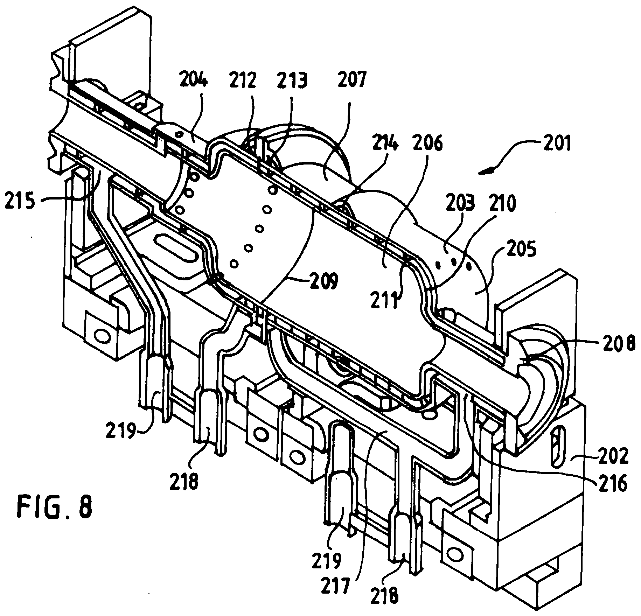

Die Blasformeinheit 201 aus Fig. 8 umfaßt ein Chassis 202 sowie ein daran befestigtes Blasformwerkzeug 203. Das Blasformwerkzeug 203 besteht aus mehreren Abschnitten 204, 205, welche in axialer Richtung hintereinander reih- bzw. stapelbar sind. Man erkennt in Fig. 8 eine rundum laufende Stoßfuge 209. Dadurch lassen sich unterschiedliche Formwerkzeuge 203 mit verschiedenen Abschnitten 204, 205 zusammensetzen.

Alternativ und/oder kumulativ kann das Blasformwerkzeug 203 auch in Längsrichtung unterteilt sein, bspw. in zwei zueinander symmetrische Halbschalen, von denen eine bspw. der Darstellung gemäß Fig. 8 entsprechen könnte. Dies hat bspw. den Vorteil, dass ein Rohling oder Extrudat zunächst in eine Streckvorrichtung eingespannt werden und vorgestreckt werden kann, bevor derartige Halbschalen zusammengefahren werden und den gestreckten Rohling sodann umschließen. Infolgedessen können auch kurze Schläuche verarbeitet werden, wobei durch die Vorstreckung sich besonders dünne Wandstärken erzielen lassen, bei gleichzeitig minimalem Ausschuß.

Das Blasformwerkzeug 203 bzw., dessen Abschnitte 204, 205 ist gebildet aus zwei zueinander kongruenten Schalen, nämlich einer inneren Schale 206 und einer äußeren Schale 207. Diese haben einen geringen Abstand von bspw. 1 bis 10 mm, vorzugsweise von 2 bis 8 mm, insbesondere von 3 bis 6 mm, und begrenzen daher - zusammen mit stirnseitigen Abschlüssen 208 - einen öder mehrere Hohlräume 210.

Diese Hohlräume 210 werden stabilisiert durch eine Vielzahl von kleinen, stiftartigen Verbindungen 211, welche sich jeweils lotrecht zwischen den beiden einander zugewandten Oberflächenabschnitten der beiden Schalen 206, 207 erstrecken. Manche oder alle dieser Stifte 211 können in ihrer Längsrichtung von je einem Kanal durchsetzt sein, durch welchen bei Bedarf ein Unterdruck an der Innenseite des Blasformwerkzeugs 203 erzeugt werden kann.

Das Blasformwerkzeug 203 ist an seiner Außenseite in verschiedenen, von seiner Längsachse etwa lotrecht durchsetzten Ebenen von je einem ringförmigen Kanal 212, 213, 214 umgeben. Diese Ringkanäle 212 - 214 kommunizieren über jeweils mehrere, entlang des Umfangs des Blasformwerkzeugs 203 verteilte Bohrungen mit dem jeweils darunter liegenden Hohlraum 209, 210. Die Ringkanäle 209, 210 umgeben das Blasformwerkzeug 203 vor allem in dessen radial erweiterten Bereichen, während in den verjüngten Bereichen ein Zu- oder Ablauf direkt angeschlossen sein kann, wie bei den Mündungen 215, 216 dargestellt.

Wie der Fig. 8 weiter zu entnehmen ist, können jeweils zwei oder mehrere Ringkanäle 212 - 214 und/oder Mündungen 215, 216 miteinander verbunden sein, bspw. durch eine etwa in Längsrichtung verlaufende Leitung 217, an welche sodann die eigentliche Zu- oder Ableitung 218, 219 angeschlossen ist. Vorzugsweise befindet sich zwischen zwei an eine Zuleitung 218 angeschlossenen Ringkanälen 212 - 214 und/oder Mündungen 215, 216 eine Ableitung 219, die über einen Ringkanal 212 - 214 und/oder eine Mündung 215, 216 an den betreffenden Hohlraum 209, 210 angeschlossen ist, so dass das Medium innerhalb eines Hohlraums 209, 210 in unterschiedlichen axialen Richtungen strömen kann.

Bevorzugt verlaufen alle Übergangsbereiche zwischen radial erweiterten und radial verjüngten Bereichen entlang sanft geschwungener Flächen.

Die Erfindung bietet den Vorteil, dass aufgrund der sehr dünnwandigen Anordnung und der guten, großflächigen, vollständigen Beobachtbarkeit der Forminnentemperatur anhand der damit fast identisch übereinstimmenden Formaußentemperatur ein vergleichsweise kleiner Temperaturgradient von bspw. 10 °C bis 12 °C oberhalb (bei Erwärmung) bzw. unterhalb (bei Abkühlung) der jeweils gewünschten Forminnentemperatur völlig ausreichend ist, um in einem kürzesten Zeitraum von einigen Sekunden die Forminnentemperatur auf ein gewünschtes Temperaturniveau zu treiben:

$$T_{Vorlauf} = T_{Form} \pm \; 2 \; [8 \text{ bis } 12\,^{\circ}C] \; 2$$

Die Vorteile dieser Maßnahme lassen sich am besten an einem bevorzugten Werkstoff für einen Ballonrohling beschreiben, wie aromatische, äther-basierte Polyurethansorten, wie z.B. "Pellethane". Dieses Material sollte zur Fixierung möglichst auf einen Temperaturbereich von etwa $T_{Form}$ = 152 bis 158 °C erhitzt und dort ausgeformt werden; zu hohe formwirksame Temperaturen lassen das fertige Produkt zu weich werden und führen zu einem partiellen Verlust der angestrebten Druck- und Formstabilität. Das Material geht bei Temperaturen über 165 Grad Celsius sukzessive in den amorphen, ungeordneten Zustand über, die durch die axiale und radiale Streckung erreichte Ausrichtung der Polymerketten geht verloren. Bei ca. 185 °C schmilzt das Material, was unbedingt vermieden werden muss.

In der Regel stellt sich ein optimales Formungsergebnis ein, wenn der aus der Form entnommene Ballon eine allseitige Retraktion von ca. 8 bis 12 % unter das Formmaß zeigt. Er weist dann die gewünschten mechanischen Charakteristika, wie Formstabilität unter steigendem Fülldruck, und mechanische Beständigkeit auf.

Optimale Ergebnisse erzielt man, wenn die Vorlauftemperatur $T_{vorlauf}$ in einem Bereich von 160 bis 165 °C gewählt wird. Dies hat im Gegensatz zu bisherigen Verfahren, wo Vorlauftemperaturen $T_{vorlauf}$ von bis zu 195 °C oder darüber erforderlich waren, den Vorteil, dass selbst bei einem zu langen Verbleiben in der Form zur bestmöglich homogenen Durchwärmung niemals die Schmelztemperatur von 185 °C erreicht oder gar überschritten werden kann, ja nicht einmal der unerwünschte Erweichungsbereich oberhalb von 165 °C.

Bei einer Fertigungssequenz werden insgesamt drei verschiedene Temperaturniveaus auf der Oberfläche der Forminnenwandung wirksam, nämlich die Vorformungstemperatur $T_{Vorformung}$, bspw. $T_{vorformung}$ = 45 °C, daneben die Ausformungstemperatur $T_{Ausformung}$, bspw. $T_{Ausformung}$ = 155 °C, und die Abkühltemperatur $T_{Abkühlung}$, bspw. $T_{Abkühlung}$ = 25 °C.

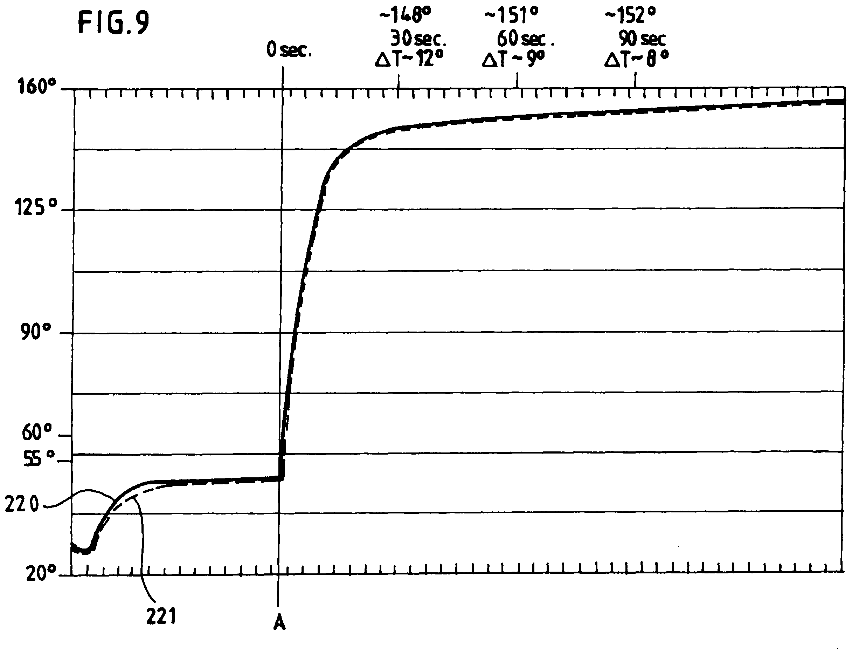

Eine typische Temperatursequenz für die Herstellung eines Ballon- oder Hohlkörpers ist in der Fig. 9 wiedergegeben, wobei außerdem zu erkennen ist, dass die Temperatur an der Innenseite der Form 203 (Kurve 220) bis auf weniger als 3 °C mit der jeweiligen Temperatur an der Außenseite der Form 203 (Kurve 221) übereinstimmt.

Wie in Fig. 9 beispielhaft gezeigt, nähert sich die wirksame Formtemperatur (gemessen auf der Forminnenseite) der Vorlauftemperatur asymptotisch, über 30 sec, 60 sec und 90 sec, bis auf wenige Grad Differenz. Infolge des dabei sehr langsamen zeitlichen Temperaturgradienten kann die Temperatur an der Forminnenseite im Idealfall auf das Grad genau eingestellt werden, insbesondere auch deshalb, weil die dortige Temperatur in fast vollkommen identischer Weise gleichzeitig an der Formaußenwand abgelesen werden kann, bspw. mit einem Infrarot-Meßgerät.

Die Formwandung besteht im entsprechenden Fall aus einem konturparallelen gesinterten Stahl-Gehäuse mit allseitiger Wandstärke von 2mm, und die Wandungen verstrebenden pin-artigen Zylindern von 3 mm Durchmesser und 5 mm Höhe, bei einer Beabstandung der Pins zueinander von ca. 10mm. Der Fluss des Mediums (flüssiges Wasser) beträgt ca. 11 l/min.

Wie weiter oben bereits angedeutet, kann ein Formwerkzeug 301 auch entlang seiner Längsachse - bspw. entlang einer vertikalen Längsebene - in zwei Hälften 302a, 302b geteilt sein, die sodann in radialer, vorzugsweise horizontaler Richtung zusammen fahrbar sind, um das Formwerkzeug 301 zu schließen, und wieder auseinander fahrbar sind, um die Form 301 wieder zu öffnen.

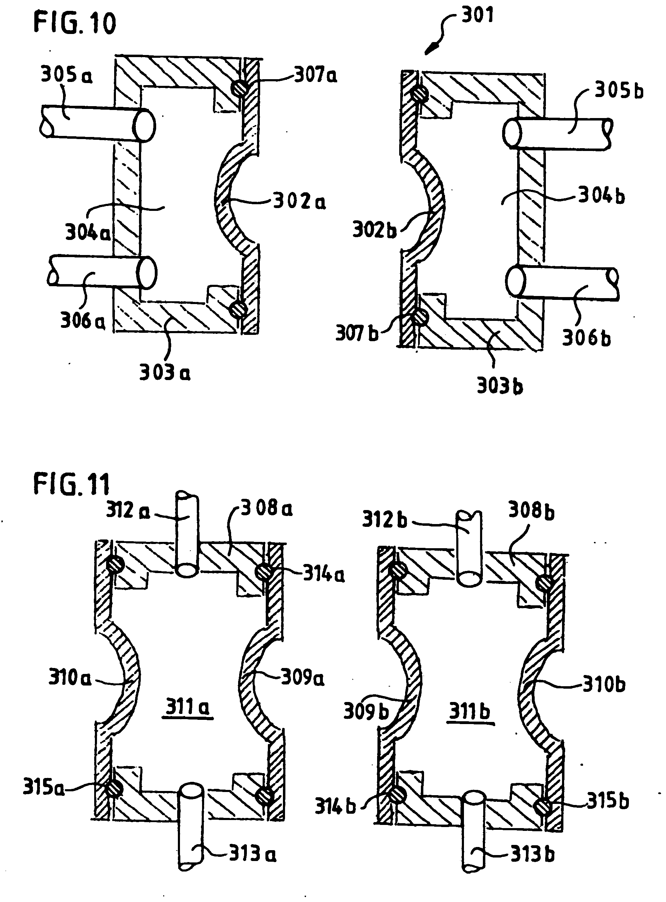

Wie Fig. 10 zeigt, sind die eigentlichen Formhälften 302a, 302b dabei jeweils lösbar an einem beweglich gelagerten Chassis 303a, 303b festgelegt, bspw. angeschraubt. Ein solches Chassis kann bspw. die Form eines einseitig offenen Trogs aufweisen, dessen offene Seite von der betreffenden Formhälfte 302a, 302b verschlossen wird. Innerhalb des dabei umschlossenen Hohlraums 304a, 304b könnte dann das Temperierungsmedium fließen, welches über Leitungen 305a, 305b, 306a, 306b zu- und abgeleitet wird. Damit dieses Medium nicht entlang der Stoßfuge zwischen Chassis 303a, 303b und, Formhälfte 302a, 302b entweichen kann, ist dazwischen je eine rundumlaufende Dichtung 307a, 307b vorgesehen, bspw. in eine rundumlaufende Nut oder Mulde eingelegt.

Die Fig. 11 zeigt eine Weiterentwicklung dieses Prinzips. Hierbei ist anstelle der Chassis 303a, 303b jeweils ein Rahmen 308a, 308b vorgesehen, welche beweglich gelagert sind, insbesondere in einer Richtung aufeinander zu bzw. voneinander weg. Dabei ist nicht nur an den einander zugewandten Innenseiten dieser beiden Rahmen 308a, 308b jeweils eine Formhälfte 309a, 309b festgelegt, sondern auch an der jeweils außen liegenden Seite ein weiteres Inlay 310a, 310b, dessen Querschnitt vorzugsweise mit der betreffenden Formhälfte 309a, 309b identisch oder dazu spiegelverkehrt ist. Wie Fig. 11 weiter zu entnehmen ist, kann das Temperierungsmedium dem von dem Rahmen 308a, 308b, der Formhälfte 309a, 309b und dem außen festgelegten Inlay 310a, 310b umschlossenen Hohlraum 311a, 311b in diesem Fall mittels den Rahmen 308a, 308b durchsetzender Leitungen 312a, 312b, 313a, 313b zu- und von diesem wieder abgeleitet werden. Damit das Temperierungsfluid nicht entweichen kann, sind sowohl die Formhälten 309a, 309b als auch die außen festgelegten Inlays 310a, 310b durch je eine rundumlaufende Dichtung 314a, 314b, 315a, 315b gegenüber dem Rahmen 308a, 308b abgedichtet.

Die Ausführungformen gemäß der Fig. 10 und 11 haben den Vorteil, dass für die Herstellung unterschiedlicher Ballons nur minimale Veränderungen an der dazu verwendeten Apparatur erforderlich sind.

Bezugszeichenliste

| | | | |
|---|---|---|---|
| 1 | Anlage | 26 | Öffnung |
| 2 | Blasformeinheit | 27 | Versorgungssäule |
| 3 | Blasformwerkzeug | 28 | Fertigungsinsel |
| 4 | Rohschlauch | 29 | Trasse |
| 5 | Streckvorrichtung | 30 | Fläche |
| 6 | Verteilerelement | 31 | Tisch |
| 7 | Ventilblock | 40 | Segment |
| 8 | Versorgungskreislauf | 41 | Kanalsystem |
| 9 | Fertigurigsbereich | 42 | Fensterung |
| 10 | Zentraleinheit | 43 | Wandaufbau |
| 11 | Spannvorrichtung | 44 | Meßpunkt |
| 12 | Segment | 101 | intrarektaler Ankerballon |
| 13 | Formungsraum | 102 | transanales Segment |
| 14 | Wandung | 103 | trichterartiges Mundstück |
| 15 | Perfusionsraum | 104 | Schlauch |
| 16 | Grenzfläche | 105 | Sammelgefäß |
| 17 | Grenzfläche | 106 | Rohschlauch |
| 18 | Verstrebung | 107 | Streckvorrichtung |
| 19 | Ausformung | 108 | Schlauchende |
| 20 | Kanal | 109 | Aufblasdorn |
| 21 | Abschnitt | 110 | Rohr |
| 22 | Kanal | 111 | Werkzeug |
| 23 | Luftauslaß , | 112 | Klemmvorrichtung |
| 24 | Drucksensor | 201 | Blasformeinheit |
| 25 | Anschluss | 202 | Chassis |
| 203 | Blasformwerkzeug | | Dichtung |
| 204 | Abschnitt | 308 | Rahmen |
| 205 | Abschnitt | 309 | Formhälfte |
| 206 | innere Schale | 310 | Inlay |
| 207 | äußere Schale | 311 | Hohlraum |
| 208 | Abschluß | 312 | Zuleitung |
| 209 | Stoßfuge | 313 | Ableitung |
| 210 | Hohlraum | 314 | Dichtung |
| 211 | stiftartige Verbindungen | 315 | Dichtung |
| 212 | Ringkanal | | |
| 213 | Ringkanal | | |
| 214 | Ringkanal | | |
| 215 | Mündung | | |
| 216 | Mündung | | |
| 217 | Verbindungsleitung | | |
| 218 | Zuleitung | | |
| 219 | Ableitung | | |
| 220 | Kurve | | |
| 221 | Kurve | | |
| 301 | Formwerkzeug | | |
| 302 | Formhälfte | | |
| 303 | Chassis | | |
| 304 | Hohlraum | | |
| 305 | Zuleitung | | |
| 306 | Ableitung | | |

EP 2 321 109 B1

**Patentansprüche**

1. Vorrichtung (1,2) zur Herstellung von Ballon- oder Hohlkörpern aus einem schlauchförmigen Rohling oder Extrudat durch Blasformung, umfassend wenigstens ein Formwerkzeug (3,203,301) mit zumindest einer temperierbaren Hohlform, innerhalb welcher der Rohling bis zu seiner gewünschten Gestalt aufgebläht und in dieser durch Erwärmung sowie anschließende Kühlung fixiert werden kann, wobei die Hohlform des Formwerkzeugs (3,203,301) als Wandung (14) ausgebildet ist mit einer nach innen und einer nach außen begrenzenden Oberfläche (16,17) des Formwerkzeugs (3,203,301), wobei sich etwa mittig zwischen diesen Oberflächen (16,17) des Formwerkzeugs (3,203,301) ein oder mehrere Hohlräume (15,20,22,210,304,311) für die Zirkulation eines flüssigen, Mediums befindet (-en), wobei der in die Werkzeugwandung integrierte medium-führende Innenraum nach außen und innen von einer Wandung jeweils identischer Stärke abgegrenzt ist, also einen symmetrischen Wandaufbau aufweist, und wobei der strukturelle Aufbau und die Abmessungen der nach innen gerichteten Wandung (16) des Formwerkzeugs (3,203,301) zu der Form seiner nach außen gerichteten Wandung (17) so weit wie möglich kongruent oder symmetrisch ist, wobei durch den symmetrischen Wandaufbau des Formwerkzeuges (3,203,301) im Verbund mit der Wiedergabe der Forminnenkontur auf der Formaußenwandung die Temperaturentwicklung auf dessen Außenseite (17) der Temperaturentwicklung auf dessen Innenseite (16) nahezu entspricht, und so die Ablesung des Temperaturverlaufs, wie er sich an der Forminnenfläche (16) darstellt, auf der Formaußenfläche (17) allseitig möglich macht, wobei an der gegenüberliegenden Fläche der Formaußenwandung (17) die unmittelbare wirksame Formungstemperatur an der Forminnenwandung (16) des Formwerkzeugs (3,203,301), wie sie auf den zu formenden Kunststoff einwirkt, z.B. durch Infrarot-Bildgebung über alle Flächen simultan, in quasi indirekter Weise, dargestellt und im Verlauf beobachtet und erfasst werden kann, insbesondere in Echtzeit.

2. Vorrichtung nach Anspruch 1, **dadurch gekennzeichnet, dass** die Wandung des Formwerkzeuges (3) einen symmetrischen Aufbau oder eine Struktur mit konstanter Stärke aufweist, der es erlaubt, die Temperaturentwicklung auf der Innenseite (16) mit der Temperatur auf der Außenwand (17) des Formwerkzeuges etwa gleichzusetzen, und so punktuell oder flächig eine indirekte präzise Ablesung der Formungstemperatur an der Grenzfläche (16) zum auszuformenden Produkt ermöglicht, wobei die derartige Wandungs-Symmetrie über die gesamte formungsrelevante Oberfläche des Formwerkzeuges (3,203,301) ausgedehnt werden kann, oder auch nur als fensterartiger Ausschnitt (42) in das Formwerkzeug (3,203,301) integriert ist.

3. Vorrichtung nach Anspruch 2, **dadurch gekennzeichnet, dass** sich die Temperatur an der Innenseite (16), d.h. an der Formungsoberfläche, von der Temperatur der Außenwand (17) des Formwerkzeugs (3,203,301) um weniger als 5 °C voneinander unterscheidet, vorzugsweise um weniger als 3 °C, insbesondere um 1 °C oder weniger.

4. Vorrichtung nach einem der vorhergehenden Ansprüche, **dadurch gekennzeichnet, dass** das Formwerkzeug (3,203,301) aus zwei zueinander konzentrischen, voneinander beabstandeten Flächen (16,17) gebildet ist, deren einander zugewandte Innenseiten einen etwa konstanten Abstand voneinander aufweisen und einen geschlossenen Hohlraum begrenzen, wobei die zwei zueinander konzentrischen, voneinander beabstandeten Flächen (16,17) bevorzugt jeweils eine Stärke aufweisen von 0,5 bis 8 mm, vorzugsweise eine Stärke von 1 bis 5 mm, insbesondere eine Stärke von 1,5 bis 3 mm.

5. Vorrichtung nach Anspruch 4, **dadurch gekennzeichnet, dass** die zwei zueinander konzentrischen, voneinander beabstandeten Flächen (16,17) ein- oder mehrmals miteinander verbunden sind, insbesondere durch ein oder mehrere stiftförmige Elemente (18,211), deren Längsachsen sich etwa lotrecht zu dem jeweils angrenzenden Bereich der beiden zueinander konzentrischen, voneinander beabstandeten Flächen (16,17) erstrecken, oder indem in der Formwandung (14) räumlich komplexe Netze von medium-führenden Kanälen (20,22) integriert sind.

6. Vorrichtung nach einem der vorhergehenden Ansprüche, **dadurch gekennzeichnet, dass** durch die geringen verwendeten Außen- und Innenwandstärken des Formwerkzeuges (3,203,301) im Verbund mit einer netz- bzw. stegartigen Verstrebung der gegenüberliegenden Formwandungen (16,17), die Masse bzw. die Wärmekapazität der Gesamtform deutlich reduziert ist und vorzugsweise kleiner ist als die Wärmekapazität des Gesamtvolumens des in den Gesamthohlraum des Formwerkzeugs (3,203,301) einfüllbaren Mediums, so dass bei der Übertragung von Wärmeenergie auf die Innenwandung (16) der Hohlform sich die Temperatur des eingefüllten Mediums kaum ändern kann bzw. das Zeitintervall bis zur Angleichung der Temperatur der Forminnenwand (16) und dem energiezuführenden oder -abführenden Medium auf wenige Sekunden verkürzt werden kann.

7. Vorrichtung nach einem der vorhergehenden Ansprüche, **dadurch gekennzeichnet, dass** ein Formwerkzeug (3,203,301) trotz der niedrigen Formmassen Druckwerten der temperierenden Medien von bis zu 25 bar widersteht.

8. Vorrichtung nach einem der vorhergehenden Ansprüche, **dadurch gekennzeichnet, dass** das einzelne Formwerkzeug (3,203,301) für die Perfusion mit einem einheitlichen Medium konzipiert ist, welches dem Prozess auf verschiedenen Temperaturniveaus, zugeführt wird, so dass die verschieden temperierten Medien vermischbar sind und nur einen einzigen Perfusionsraum (15) erfordern.

9. Vorrichtung (1,2) nach einem der vorhergehenden Ansprüche, **dadurch gekennzeichnet, dass** eine Klemmvorrichtung (112) vorgesehen ist zum Festklemmen der Enden (108) des zu verformenden Schlauchs (106), durch wenigstens eine Streckvorrichtung (107), um den Abstand zwischen den Klemmvorrichtungen (112) zu erhöhen, sowie durch wenigstens einen Aufblasdorn (109) innerhalb eines Schlauchs (106) im Bereich einer Klemmvorrichtung (112) vorgesehen ist, um den Schlauch (106) mittels Überdruck aufzudehnen.

10. Verfahren zur Herstellung von Ballon- oder Hohlkörpern aus einem vorextrudierten, schlauchförmigen Rohling oder Extrudat durch Blasformung, wobei der Rohling innerhalb zumindest einer temperierbaren Hohlform wenigstens eines Formwerkzeugs (3,203,301) zu seiner gewünschten Gestalt aufgebläht und in dieser durch Erwärmung sowie anschließende Kühlung fixiert wird, wobei eine Hohlform des Formwerkzeugs (3;203;301) verwendet wird, die als Wandung (14) ausgebildet ist mit einer nach innen und einer nach außen begrenzenden Oberfläche (16,17) des Formwerkzeugs (3;203;301), wobei ein flüssiges Medium durch ein oder mehrere Hohlräume (15,20,22,210,304,311) zirkuliert, der/die sich etwa mittig zwischen diesen Oberflächen (16,17) des Formwerkzeugs (3;203;301) befindet (-en), wobei der in die Werkzeugwandung integrierte medium-führende Innenraum nach außen und Innen von einer Wandung jeweils identischer Stärke abgegrenzt ist, also einen symmetrischen Wandaufbau aufweist, und wobei der strukturelle Aufbau und die Abmessungen der nach innen gerichteten Wandung (16) des Formwerkzeugs (3;203;301) zu der Form seiner nach außen gerichteten Wandung (17) so weit wie möglich kongruent oder symmetrisch ist, wobei durch den symmetrischen Wandaufbau des Formwerkzeuges (3;203;301) im Verbund mit der Wiedergabe der Forminnenkontur auf der Formaußenwandung die Temperaturentwicklung auf dessen Außenseite (17) der Temperaturentwicklung auf dessen Innenseite (16) nahezu entspricht, und so die Ablesung des Temperaturverlaufs, wie er sich an der Forminnenfläche (16) darstellt, auf der Formaußenfläche (17) allseitig möglich macht, wobei an der gegenüberliegenden Fläche der Formaußenwandung (17) die unmittelbare wirksame Formungstemperatur an der Forminnenwandung (16) des Formwerkzeugs (3;203;301), wie sie auf den zu formenden Kunststoff einwirkt, z. B. durch Infrarot-Bildgebung über alle Flächen simultan, in quasi indirekter Weise, dargestellt und im Verlauf beobachtet und erfasst wird, insbesondere in Echtzeit.

11. Verfahren gemäß Anspruch 10, **dadurch gekennzeichnet, dass** ein vor-extrudierter Rohschlauch (106), vorzugsweise aus Polyurethan, PVC und/oder LDPE, in einer einseitig oder beidseitig angeordneten Streckvorrichtung (107). an beiden Schlauchenden (108) fixiert wird; und über den axial wirkenden Zugmechanismus (107) der Schlauch (106) in Längsrichtung gedehnt wird; und wobei das Schlauchlumen wird über entsprechende Aufblasdorne (109), über welche der Rohschlauch (106) zugfest geklemmt ist, im Anschluss oder zeitgleich mit der axialen Streckung mit hohem Druck beaufschlagt wird, so dass sich der Rohschlauch (106) zusätzlich zur axialen Streckung auch radial aufdehnt.

12. Verfahren nach einem der Ansprüche 10 oder 11, **dadurch gekennzeichnet, dass** die jeweils im Werkzeug (3) zu erreichende Temperatur durch Fluten von medium-führenden Hohlräumen (15,20,22,210,304,311) mit einem Medium definierter Temperatur ($T_{Vorlauf}$) direkt, präzise, mit nur geringer zeitlicher Verzögerung sowie einem technisch und regeltechnisch insgesamt niedrigen Aufwand, erreicht wird.

13. Verfahren nach Anspruch 12, **dadurch gekennzeichnet, dass** sich aufgrund der sehr dünnwandigen Anordnung die Temperatur ($T_{Vorlauf}$) des Temperierungsmediums von dem gewünschten Temperaturniveau ($T_{Form}$) an der Forminnenseite (16) nach einem kürzesten Zeitraum von einigen Sekunden gemäß der folgenden Formel unterscheidet:

$$T_{Vorlauf} = T_{Form} \pm 2 \; [8 \text{ bis } 12] \; 2$$

~~8...12~~ °C,

d.h., nach einem kürzeten Zeitraum von einigen Sekunden verbleibt ein kleiner Temperaturgradient zwischen der Forminnentemperatur ($T_{Form}$) und der Medientemperatur ($T_{Vorlauf}$).

14. Verfahren nach einem der Ansprüche 10 bis 13, **dadurch gekennzeichnet, dass** beim Blasformen sämtliche Segmente bzw. formenden Oberflächen des verwendeten Formwerkzeugs (3;203;301) homogen, exakt kontrollierbar und zeitsynchron erwärmt werden.

**Claims**

1. Device (1,2) for the production of balloon or hollow parts starting from a tubular molding blank or extruded parent material by blow molding comprising at least one molding tool (3,203,301) with at least one hollow mold with controllable temperature, inside of which the molding blank is expanded up to its desired shape, and inside of which it can be fixated by heating followed by subsequent cooling, where the hollow mold of the molding tool (3,203,301) is formed as a walling (14) having a surface that delimits it to the inside as well as to the outside (16,17), and where approximately centered in between these surfaces (16,17) of the molding tool (3,203,301) one or more cavities (15, 20, 22, 210, 304, 311) for the circulation of a liquid medium are located, and, where the medium-conducting internal space integrated inside the molding tool walling is delimited to the inside and to the outside by a walling of approximately identical thickness, thus exhibiting a symmetric wall structure, and where the structural composition and the dimensions of the walling (16) of the molding tool (3,203,301) directed to the inside are to the possible extent congruent with or symmetrical to the shape of its walling (17) directed to the outside, and where because of the symmetrical wall structure of the molding tool (3,203,301) in conjunction with the reproduction of the inner mold contour at the outer side of the mold walling the temperature development at its outer surface (17) approximately corresponds to the temperature development at its inner side (16), and therefore enables a reading of the temperature profile as it develops at the inner surface (16) at the outer surface (17) of the mold on all sides, and where at the opposite surface of the outer walling (17) of the mold the directly effective shaping temperature at the inner walling (16) of the molding tool (3,203,301), as it acts upon the plastic material to be shaped, can be shown, observed in its course, and captured, e.g. by infrared imaging simultaneously over all surfaces, in a quasi-indirect way, and that particularly in real-time.

2. Device according to claim 1, **characterized by** the fact that the walling of the molding tool (3) features a symmetrical structure or a structure of constant thickness that allows to approximately equate the temperature development at the inner side (16) with the temperature at the outer wall (17) of the molding tool (3) and so to enable a selective or over an area indirect precise reading of the shaping temperature at the boundary surface (16) to the product to be shaped, whereat such type of walling symmetry can be extended over the entire shaping-relevant surface of the molding tool (3,203,301), or also may be just integrated as a window-like cutout (42) in the molding tool (3,203,301).

3. Device according to claim 2, **characterized by** the fact that the temperature at the inner side (16), i.e. at the shape forming surface, differs from the temperature at the outer wall surface (17) of the molding tool (3,203,301) by less than 5 °C, preferably by less than 3 °C, particularly by 1 °C or less.

4. Device according to one of the foregoing claims, **characterized by** the fact that the molding tool (3,203,301) is comprised of two, to each other concentric, surfaces (16,17) with a clearance in-between, where the respective inner sides of which facing each other exhibit an approximately constant distance to each other and delimit a closed hollow space, at which the two, to each other concentric, surfaces (16,17) each mainly exhibit a thickness of 0.5 to 8 mm, preferably a thickness of 1 to 5 mm, in particular a thickness of 1.5 to 3 mm.

5. Device according to claim 4, **characterized by** the fact that the two, to each other concentric, surfaces (16,17) with a clearance in-between are connected to each other at one or several points, particularly by one or more pin-shaped elements (18,211) whereat their longitudinal axes extend approximately perpendicular to the respective bordering areas of the both, to each other concentric, surfaces (16,17) with a clearance in-between, or by having spatially complex nets of medium-conducting channels integrated into the mold walling (14).

6. Device according to one of the foregoing claims, **characterized by** the fact that due to the small outer and inner wall thicknesses applied at the molding tool (3,203,301) in conjunction with a net-like or web-type strutting with the respective opposite mold walling (14,16) the mass or the overall heat capacity of the entire mold is significantly reduced, so that preferably the same is less than the heat capacity of the total volume of medium that can be filled into the aggregate hollow space of the molding tool (3,203,301) so that the temperature of the filled-in medium can hardly change due to the thermal energy transfer to the inner walling (16) of the hollow mold, or so that the time interval until an equalization of the temperature at the inner surface (16) and the temperature of the energy supplying or dissipating medium can be reduced to a few seconds.

7. Device according to one of the foregoing claims, **characterized by** the fact that a molding tool (3,203,301) withstands the pressure values of the temperature regulating medium of up to 25 bar in spite of the low masses of the mold.

8. Device according to one of the foregoing claims, **characterized by** the fact that the individual molding tool (3,203,301) is designed for the perfusion with a uniform medium, which is brought into the process at different temperature levels so that the varying temperature regulating media are intermixable and require only one single perfusion space.

9. Device (1,2) according to one of the foregoing claims, **characterized by** the fact that a clamping fixture (112) is provided for clamping tight the ends (108) of the hose (106) to be deformed by at least one plug connector (107) in order to increase the distance between the clamping fixtures (112), as well as by at least one blowup mandrel provided inside a hose (106) near one clamping fixture (112) for expanding the hose (106) by action of excess pressure.

10. Method for the production of balloon or hollow parts starting from a pre-textured tubular molding blank or extruded parent material by blow molding, in which the molding blank is expanded inside of, at the minimum, one temperature-regulated hollow mold of at least one molding tool (3,203,301) to the desired shape, and inside of which it can be fixated by heating followed by subsequent cooling, whereby the hollow mold of the molding tool (3,203,301) used is formed as a walling (14) having a surface that delimits it to the inside as well as to the outside (16,17), and where a liquid medium circulates through one or more cavities (15, 20, 22, 210, 304, 311) that are located approximately centered between these surfaces (16,17) of the molding tool (3,203,301), and where the medium-conducting internal space integrated inside the molding tool walling is delimited to the inside and to the outside by a walling of approximately identical thickness, thus exhibiting a symmetric wall structure, and where the structural composition and the dimensions of the walling (16) of the molding tool (3,203,301) directed to the inside are to the possible extent congruent with or symmetrical to the shape of its walling (17) directed to the outside, and where because of the symmetrical wall structure of the molding tool (3,203,301) in conjunction with the reproduction of the inner mold contour at the outer side of the mold walling the temperature development at its outer surface (17) approximately corresponds to the temperature development at its inner side (16), and therefore enables a reading of the temperature profile as it develops at the inner surface (16) at the outer surface (17) of the mold on all sides, and where at the opposite surface of the outer walling (17) of the mold the directly effective shaping temperature at the inner walling (16) of the molding tool (3,203,301), as it acts upon the plastic material to be shaped, can be shown, observed in its course, and captured, e.g. by infrared imaging simultaneously over all surfaces, in a quasi-indirect way, and that particularly in real-time.

11. Method according to claim 10, **characterized by** the fact that a pre-extruded basic raw hose (106), preferably made of polyurethane, PVC, and/or LDPE, is fixated in a stretching fixture (107) arranged at one or both ends of the hose (108), and where the hose (106) is stretched in longitudinal direction by the axially acting pulling mechanism (107), and in which the volume of the hose (106) is tightly clamped-in at two corresponding blowup mandrels (109) over which the basic raw hose (106) is charged with pressure, either simultaneously with the stretching process or thereafter, so that the basic raw hose (106) also expands radially in addition to the axial stretching.

12. Method according to one of the claims 10 or 11, **characterized by** the fact that the temperature to be reached inside the molding tool (3) by flooding the medium-conducting internal cavities (15, 20, 22, 210, 304, 311) with a medium of defined temperature ($T_{Vorlauf}$) is directly and precisely achieved with only little temporal delay and with altogether low technical and control technological effort.

13. Method according to claim 12, **characterized by** the fact that because of the thin-wall design the temperature ($T_{Vorlauf}$) of the temperature controlling medium differs from the desired temperature level ($T_{Form}$) at the inner side (16) of the mold after a shortest-possible time interval of a few seconds according to the following formula:

$$(T_{Vorlauf}) = (T_{Form}) \pm 8 \text{ to } 12 \ °C,$$

i.e. after a shortest-possible time interval of a few seconds a small temperature gradient between inside temperature of the mold ($T_{Form}$) and temperature of the medium ($T_{Vorlauf}$) will remain.

14. Method according to one of the claims 10 through 13, **characterized by** the fact that during blow molding all segments respectively shaping surfaces of the molding tool (3,203,301) are heated homogeneously, exactly controllable and with chronological synchronism.

## Revendications

1. Dispositif (1, 2) de production de corps de ballonnet ou de corps creux à partir d'une ébauche ou d'un extrudat tubulaire par moulage par soufflage, comportant au moins un outil de moulage (3, 203, 301) avec au moins un moule creux thermorégulable à l'intérieur duquel l'ébauche est gonflée jusqu'à obtention de la forme souhaitée et qui peut être fixée dans ledit moule creux par augmentation puis par abaissement de la température, en ce que le moule creux de l'outil de moulage (3, 203, 301) se présente sous forme de paroi (14) avec une surface de délimitation (16, 17) vers l'intérieur et vers l'extérieur de l'outil de moulage (3, 203, 301), en ce qu'une ou plusieurs cavités (15, 20, 22, 210, 304, 311) sont situées approximativement au centre entre ces surfaces (16, 17) de l'outil de moulage (3, 203, 301) pour permettre la circulation d'un milieu liquide, en ce que l'espace intérieur intégré guidant le milieu dans la paroi de moule est délimité vers l'extérieur et l'intérieur par une paroi respectivement d'épaisseur identique qui présente donc une construction de paroi symétrique, et en ce que la construction structurelle et les dimensions de la paroi orientée vers l'intérieur (16) de l'outil de moulage (3, 203, 301) est dans la mesure du possible congruente ou symétrique à la forme de sa paroi orientée vers l'extérieur (17), en ce que par la construction de paroi symétrique de l'outil de moulage (3, 203, 301) en association avec la reproduction du contour intérieur de moule sur la paroi extérieure de moule l'évolution de la température sur sa face extérieure (17) correspond pratiquement à l'évolution de la température sur sa face intérieure (16), permettant ainsi de tous les côtés la lecture de la courbe de température sur la surface extérieure du moule (17), comme elle se présente au niveau de la surface intérieure de moule (16), en ce qu'au niveau de la surface opposée de la paroi extérieure du moule (17) la température de moulage efficace directe sur la paroi intérieure du moule (16) de l'outil de moulage (3, 203, 301), comme elle agit sur la matière plastique à mouler, par exemple comme elle peut être représentée par imagerie infrarouge simultanément sur toutes les surfaces, d'une manière quasi indirecte, et observée pendant l'opération et enregistrée, en particulier en temps réel.

2. Dispositif selon la revendication 1, **caractérisé en ce que** la paroi de l'outil de moulage (3) présente une construction symétrique ou une structure d'épaisseur constante qui permet d'égaler approximativement l'évolution de la température sur la face intérieure (16) à la température sur la paroi extérieure (17) de l'outil de moulage permettant ainsi de manière ponctuelle ou surfacique une lecture précise indirecte de la température de moulage au niveau de la surface limite (16) par rapport au produit qui doit être moulé, **en ce qu'**une telle symétrie de paroi peut s'étendre sur l'ensemble de la surface pertinente pour le moulage de l'outil de moulage (3, 203, 301) ou est intégrée aussi uniquement comme découpe en forme de fenêtre (42) dans l'outil de moulage (3, 203, 301).

3. Dispositif selon la revendication 2, **caractérisé en ce que** la température au niveau de la face intérieure (16), c.-à-d au niveau de la surface de moulage, diffère de la température de la paroi extérieure (17) de l'outil de moulage (3, 203, 301) de moins de 5 °C, de préférence de moins de 3 °C, en particulier de moins de 1° C.

4. Dispositif selon l'une des revendications précédentes, **caractérisé en ce que** l'outil de moulage (3, 203, 301) est formé à partir de deux surfaces (16, 17) écartées l'une de l'autre, concentriques l'une par rapport à l'autre, dont les faces intérieures tournées l'une vers l'autre présentent une distance entre elles approximativement constante et délimitent une cavité fermée, **en ce que** les deux surfaces (16, 17) écartées l'une de l'autre, concentriques l'une par rapport à l'autre présentent avantageusement respectivement une épaisseur de 0,5 à 8 mm, de préférence une épaisseur de 1 à 5 mm, en particulier une épaisseur de 1,5 à 3 mm.

5. Dispositif selon la revendication 4, **caractérisé en ce que** les deux surfaces (16, 17) écartées l'une de l'autre, concentriques l'une par rapport à l'autre sont reliées une fois ou plusieurs fois entre elles, en particulier par un ou plusieurs éléments en forme de tige (18, 211) dont les axes longitudinaux s'étendent approximativement perpendiculairement à la zone respectivement adjacente des deux surfaces (16, 17) écartées l'une de l'autre, concentriques l'une par rapport à l'autre, ou en intégrant dans la paroi de moule (14) des réseaux spatialement complexes de canaux (20, 22) conduisant le fluide.

6. Dispositif selon l'une des revendications précédentes, **caractérisé en ce que** par les faibles épaisseurs de parois extérieures et intérieures utilisées de l'outil de moulage (3, 203, 301) en association avec une stabilisation réticulaire ou en forme de nervure des parois de moule (16, 17) opposées, la masse et/ou la capacité thermique du moule total est considérablement réduite et de préférence est inférieure à la capacité thermique du volume total du fluide pouvant être rempli dans la cavité totale de l'outil de moulage (3, 203, 301) de telle sorte que lors du transfert thermique d'énergie thermique sur la paroi intérieure (16) du moule creux la température du fluide rempli ne peut à peine varier et/ou l'intervalle de temps jusqu'à ajustement de la température de la paroi intérieure du moule (16) et du fluide alimentant ou évacuant de l'énergie peut être réduit à quelques secondes.

7. Dispositif selon l'une des revendications précédentes, **caractérisé en ce qu'**un outil de moulage (3, 203, 301) résiste à des valeurs de pression des milieux thermorégulés allant jusqu'à 25 bar malgré les faibles matières à mouler.

8. Dispositif selon l'une des revendications précédentes, **caractérisé en ce que** l'outil de moulage (3, 203, 301) individuel est conçu pour la perfusion avec un milieu uniforme qui est fourni au processus à des niveaux de température différents de telle sorte que les milieux différemment thermorégulés peuvent être mélangés et ne nécessitent qu'un seul espace de perfusion (15).

9. Dispositif (1, 2) selon l'une des revendications précédentes, **caractérisé en ce qu'**un dispositif de serrage (112) est prévu pour le serrage des extrémités (108) du tuyau flexible (106) à façonner par au moins un dispositif d'étirage (107) pour augmenter la distance entre les dispositifs de serrage (112) ainsi que par au moins un mandrin de soufflage (109) dans un tuyau flexible (106) dans la zone d'un dispositif de serrage (112) pour étirer le tuyau flexible (106) par surpression.

10. Procédé de production de corps de ballonnet ou de corps creux à partir d'une ébauche ou d'un extrudat tubulaire préextrudé par moulage par soufflage, en ce que l'ébauche dans au moins un moule creux thermorégulable d'au moins un outil de moulage (3, 203, 301) est gonflée à sa forme souhaitée et est fixée dans ledit moule par augmentation puis par abaissement de la température, en ce qu'un moule creux de l'outil de moulage (3, 203, 301) est utilisé qui se présente sous forme de paroi (14) avec une surface de délimitation (16, 17) vers l'intérieur et vers l'extérieur de l'outil de moulage (3, 203, 301), en ce qu'un milieu liquide circule à travers une ou plusieurs cavités (15, 20, 22, 210, 304, 311), la ou les cavités étant situées approximativement au centre entre ces surfaces (16, 17) de l'outil de moulage (3, 203, 301) ; en ce que l'espace intérieur intégré guidant le milieu dans la paroi de moule est délimité vers l'extérieur et l'intérieur par une paroi respectivement d'épaisseur identique qui présente donc une construction de paroi symétrique, et en ce que la construction structurelle et les dimensions de la paroi orientée vers l'intérieur (16) de l'outil de moulage (3, 203, 301) est dans la mesure du possible congruente ou symétrique à la forme de sa paroi orientée vers l'extérieur (17), en ce que par la construction de paroi symétrique de l'outil de moulage (3, 203, 301) en association avec la reproduction du contour intérieur de moule sur la paroi extérieure de moule l'évolution de la température sur sa face extérieure (17) correspond pratiquement à l'évolution de la température sur sa face intérieure (16), permettant ainsi de tous les côtés la lecture de la courbe de température sur la surface extérieure du moule (17), comme elle se présente au niveau de la surface intérieure de moule (16), en ce qu'au niveau de la surface opposée de la paroi extérieure du moule (17) la température de moulage efficace directe sur la paroi intérieure du moule (16) de l'outil de moulage (3, 203, 301), comme elle agit sur la matière plastique à mouler, par exemple comme elle est représentée par imagerie infrarouge simultanément sur toutes les surfaces, d'une manière quasi indirecte, et observée pendant l'opération et enregistrée, en particulier en temps réel.

11. Procédé selon la revendication 10, **caractérisé en ce qu'**un tuyau flexible brut (106) préextrudé, de préférence en polyuréthane, PVC et/ou LDPE , dans un dispositif d'étirage (107) disposé d'un côté ou des deux côtés, est fixé aux deux extrémités (108) du tuyau flexible ; et le tuyau flexible (106) est étiré dans le sens longitudinal par le biais d'un mécanisme de traction (107) agissant axialement ; et **en ce que** la lumière du tuyau flexible par l'intermédiaire de mandrins de soufflage correspondants (109), par le biais desquels le tuyau flexible brut (106) est serré de manière résistante à la traction, est exposée à haute pression suivant ou en même temps que l'extension axiale de telle sorte que le tuyau flexible brut (106) s'étend non seulement dans l'extension axiale mais aussi radiale.

12. Procédé selon l'une des revendications 10 ou 11, **caractérisé en ce que** la température qui doit être atteinte respectivement dans le moule (3) par injection de cavités (15, 20, 22, 210, 304, 311) guidant le milieu, est atteinte. directement et exactement avec un milieu de température définie ($T_{Vorlaut}$), avec seulement une temporisation minime ainsi qu'une mise en œuvre technique et de régulation dans l'ensemble peu poussée.

13. Procédé selon la revendication 12, **caractérisé en ce qu'**en raison de la disposition à paroi très mince la température ($T_{Vorlauf}$) du milieu de thermorégulation diffère du niveau de température souhaité ($T_{Form}$) au niveau de la face intérieure du moule (16) après une période très courte de quelques secondes selon la formule suivante :

$$T_{Vorlauf} = T_{Form} \pm 8 \text{ à } 12 \text{ °C,}$$

c-à-d. qu'après une période très courte de quelques secondes un faible gradient de température reste entre la température intérieure du moule ($T_{Form}$) et la température du milieu ($T_{Vorlauf}$).

14. Procédé selon l'une des revendications 10 à 13, **caractérisé en ce que** lors du moulage par soufflage tous les segments et/ou les surfaces à mouler de l'outil de moulage utilisé (3, 203, 301) sont chauffés de manière homogène, peuvent être contrôlés avec précision et synchrone dans le temps.

FIG. 1a

# FIG.1b

EP 2 321 109 B1

# FIG.1c

EP 2 321 109 B1

FIG.2a

FIG.2b

FIG.3a

FIG.3b

FIG.3c

EP 2 321 109 B1

FIG.4a

42     40, 44

43                                    43

40          40

41                        43

41

FIG.4b

42          42          42

FIG. 5

FIG.6

FIG.7

**FIG. 8**

# FIG.9

EP 2 321 109 B1

## FIG. 10

## FIG. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1142689 A
- US 5993721 A

- JP H04351526 A
- US 2222461 A